# EUROPEAN PATENT SPECIFICATION

(11) **EP 4 216 169 B1**
(45) Date of publication and mention of the grant of the patent: **12.03.2025**
(21) Application number: 23152775.5
(22) Date of filing: 20.01.2023
(51) Int. Cl.: G06T 17/20, G06F 30/23, G06F 111/10, G06F 119/14

(54) **CRITICAL POINTS FOR TEST DATA PREPROCESSING**
KRITISCHE PUNKTE ZUR TESTDATENVORVERARBEITUNG
POINTS CRITIQUES POUR PRÉTRAITEMENT DE DONNÉES DE TEST

(30) Priority: 21.01.2022 US 202217580695
(43) Date of publication of application: 26.07.2023
(73) Proprietor: Dassault Systemes Americas Corp., Waltham, MA 02451 (US)
(72) Inventor: McLendon, Wesley, Johnston (US); Stewart, Joshua, Mason (US)
(74) Representative: Bandpay & Greuter

(56) References cited:
- "INTRODUCTION TO APPLIED STATISTICAL SIGNAL ANALYSIS:GUIDE TO BIOMEDICAL AND ELECTRICAL ENGINEERING APPLICATIONS", REFEREX, 31 December 2007 (2007-12-31), XP040425653
- DOERR BENJAMIN ET AL: "Detecting structural breaks in time series via genetic algorithms", SOFT COMPUTING, SPRINGER BERLIN HEIDELBERG, BERLIN/HEIDELBERG, vol. 21, no. 16, 23 February 2016 (2016-02-23), pages 4707 - 4720, XP036290223, ISSN: 1432-7643, [retrieved on 20160223], DOI: 10.1007/S00500-016-2079-0

## Description

### FIELD OF THE INVENTION

This disclosure relates to the field of data processing and more specifically relates to critical points in test data preprocessing (e.g., for material model calibration).

### BACKGROUND

Many products (e.g., tires, plastic cups, polymer membranes, etc.) deform when exposed to a load. The amount that a particular product will deform depends, at least in part, on the stiffness of the material that constitutes the part. A material model can be a virtual representation of an equation that describes the relationship between various material characteristics (e.g., applied force (or "stress") and resulting deformation (or "strain")) for a particular material. Material models can be calibrated to better represent particular materials. It is desirable that such material models be accurate."Introduction to Applied Statistical Signal Analysis Guide to Biomedical and Electrical Engineering Applications A volume in Biomedical Engineering", Third Edition, 2007, provides relevant prior art.

### SUMMARY OF THE INVENTION

The appended claims define the scope of protection. Any examples and technical descriptions of apparatuses, products and/or methods in the description and/or drawings not covered by the claims are presented not as embodiments of the invention but as background art or examples useful for understanding the invention.

In one aspect, a computer-implemented method includes receiving, in computer memory, a first test data set that comprises results of a real-world test of a material, where the first test data set comprises a plurality of test data points. The method further includes identifying one or more critical points among the test data points in the first test data set, and processing the first test data set with a computer processor to produce a second test data set with test data points that are more suitable for exercising a material model than the first test data set, wherein the second test data set includes all the test data points that were identified as critical points in the first test data set and at least some other data points. In some, but not necessarily all, instances, the second test data set has fewer test data points than the first test data set.

In another aspect, a computer-based system includes a computer processor and computer-based memory operatively coupled to the computer processor. The computer-based memory stores computer-readable instructions that, when executed by the computer processor, cause the computer-based system to: receive, in computer memory, a first test data set that comprises results of a real-world test of a material, where the first test data set comprises a plurality of test data points; identify one or more critical points among the test data points in the first test data set; and process the first test data set with a computer processor to produce a second test data set with test data points that are more suitable for exercising a material model than the first test data set, wherein the second test data set includes all the test data points that were identified as critical points in the first test data set and at least some other data points.

In yet another aspect, a non-transitory computer readable medium having stored thereon computer-readable instructions that, when executed by a computer-based processor, cause the computer-based processor to: receive, in computer memory, a first test data set that comprises results of a real-world test of a material, where the first test data set comprises a plurality of test data points; identify one or more critical points among the test data points in the first test data set; and process the first test data set with a computer processor to produce a second test data set with test data points that are more suitable for exercising a material model than the first test data set, wherein the second test data set includes all the test data points that were identified as critical points in the first test data set and at least some other data points.

In some implementations, one or more of the following advantages are present.

For example, in various implementations, the systems and techniques disclosed herein permit analysts to quickly and simply apply data filters to test data in a way that that preserves important characteristics of the data series as opposed to having to manually partition the data into windows, for example, accounting for numerous discontinuities for every filtering action that is performed on the dataset.

Additionally, material models can be useful in a wide variety of product design processes including, for example, those involving finite element analysis (FEA). FEA is a process whereby a computer, for example, simulates and/or assesses the behavior of a part or assembly under a particular set of operational or environmental conditions. FEA may be based on any one or more of a variety of different types of input data. In an exemplary implementation, FEA takes into account, as inputs, the geometry of a particular part (which may come from a computer-aided design, CAD, representation of the part), load and boundary conditions for the part, and material model(s) (representing material behavior). Accurate FEA can reduce the need to produce physical prototypes, for example, while allowing for optimization of components as part of the design process for products or assemblies. Accuracy of the FEA analysis is enhanced, of course, by enhancing the accuracy of any underlying material model(s), which the systems and techniques disclosed herein can facilitate.

Other features and advantages will be apparent from the description and drawings, and from the claims.

### BRIEF DESCRIPTION OF THE DRAWINGS

FIG. 1 is a schematic representation of an exemplary computer.
FIG. 2 is a schematic representation of an exemplary system for generating and utilizing highly accurate calibrated material models.
FIG. 3 is a flowchart that represents an implementation of a process for producing a calibrated material model that may be used, for example, for design analysis.
FIG. 4 shows a screenshot from a version of 3DEXPERIENCE^{®} Material Calibration application, adapted according to the systems and techniques disclosed herein.
FIG. 5A is a screenshot showing an example of how critical points that have been identified by a computer-implemented critical points identifier may be visually denoted on a computer display.
FIGS. 5B(1) and 5B(2) are partial views of an exemplary screenshot that enables a user to specify criteria for critical points identification / flagging.
FIG. 5C(1) and 5C(2) show screenshots illustrating that a user can directly click on data points in a plot to highlight corresponding entries in a table where the checkbox can be toggled to facilitate quick visual-based specification of critical points.
FIG. 6 is a flowchart that represents an exemplary implementation of test data preprocessing in a manner that preserves any of the critical points identified by the critical points identifier and/or refined by a user.
FIG. 7 shows an example of a portion of a test data set that undergoes regularization, noting the mapping (e.g., logical linking in computer memory) between critical points in the test data set before and after the regularization.
FIGS. 8A and 8B are screenshots that show a data set before decimation (FIG. 8A) and after decimation (FIG. 8B), where critical points (e.g., identified by a critical points identifier) are preserved from FIG. 8A to FIG. 8B even though the data set is otherwise decimated.
FIG. 9 shows a plot of nominal stress vs time in a data set that results from a decimator performing decimation while preserving any critical points.
FIG. 10 shows a plot of test data that has been decimated without flagging or preserving critical points.
FIG. 11 shows a plot of nominal stress vs time test data that has been decimated without flagging or preserving critical points bolsters this point.
FIGS. 12 and 13 are plots showing a data set that has been regularized while preserving critical points.
FIGS. 14 and 15 are plots showing a data set that has been regularized without flagging or preserving critical points.
FIGS. 16 and 17 are plots showing results of smoothing, with critical points preserved.
FIGS. 18, 19 and 20 are plots showing results of smoothing data without flagging and preserving critical points.

Like reference characters refer to like elements.

### DETAILED DESCRIPTION

This document uses a variety of terminology to describe its inventive concepts. Unless otherwise indicated, the following terminology, and variations thereof, should be understood as having meanings that are consistent with what follows.

A "material model" is representation, virtual or otherwise, of an equation that describes the relationship between various material characteristics (e.g., applied force (or "stress") and resulting deformation (or "strain")) for a particular material.

For example, the phrase "critical point" or the like refers to a point in a data set that has been identified as a data point to be preserved throughout data processing (also referred to as "preprocessing"), which may include, for example, decimation, regularization, and/or smoothing, to be applied prior to material model calibration.

A "C1 discontinuity" is a discontinuous jump in the tangent, or first derivative, of a curve.

The phrase "processor" or the like refers to any one or more computer-based processing devices. A computer-based processing device is a physical component that can perform computer functionalities by executing computer-readable instructions stored in memory.

The phrase "memory" or the like refers to any one or more computer-based memory devices. A computer-based memory device is a physical component that can store computer-readable instructions that, when executed by a processor, results in the processor performing associated computer functionalities.

Tools that handle time-series test data manipulation sometimes utilize filters and/or "windowing" to address issues of discontinuities. However, these approaches are based more on operating on a continuous signal of data and avoiding the introduction of high-frequency transients when performing steps like Fast Fourier Transforms, and do not generally address or include the idea of marking discontinuities for the purpose of preserving them in the data and automatically defining windows over which filters can operate. In some sense, the systems and techniques disclosed herein represent a convenient way to quickly define many "windows" at the dataset level that can impact the behavior of one or more filters of different kinds in an easy-to-use manner.

For cyclic data with many discontinuities, prior approaches to account for the discontinuities have been slow and tedious processes. Additionally, existing options to retain reversals in decimation-type filtering/pre-processing may be made obsolete by the more general and broadly applicable capabilities offered by the systems and techniques disclosed herein which allow the user to define a set of more general critical points, with ease, which will be retained and that could include reversals, some other sort of discontinuity, or other data.

Among the more commonly used tools for the acquisition and manipulation of test data is LabView^{™} by National Instruments. It provides "windowing" for imposing a filter on only a certain part of the data but does not provide the ability to define a set of datapoints that will be used to automatically partition the data into a large number of windows for a variety of filters being applied to the data.

WellTest^{™} software product by HIS Markit provides the ability to tag "shut-in" points in oil well time history data. The data is then treated in a piecewise manner between shut-in events when applying filters. However, this functionality is highly specific to oil well shut-ins, while the systems and techniques disclosed herein are more broadly applicable to any sorts of discontinuities in general test data. Additionally, the WellTest^{™} software does not modify the global behavior of test data pre-processing tools as do the systems and techniques disclosed herein.

In academia, the subject of data smoothing in the presence of discontinuities is an ongoing area of research. Take for example the paper by Lee (Lee, T. C. M. (2002), Automatic smoothing for discontinuous regression functions, Statistica Sinica, 12(3), 823-842.). In this work, points on which to preserve discontinuities for smoothed piecewise spline fits to noisy discontinuous data are automatically identified through the use of various optimization algorithms. However, this and associated work only addresses the use of these data points in the context of smoothing via spline fitting and differs from the systems and techniques disclosed herein in other ways as well. Another more recent work in this field by Doerr et al. (Doerr, B., Fischer, P., Hilbert, A., & Witt, C. (2017), Detecting structural breaks in time series via genetic algorithms, Soft Computing, 21(16), 4707-4720) addresses the matter of identifying what Doerr refer to as "structural breaks" in time series data for generic time data series using genetic optimization algorithms. However, this work does not go into depth regarding the application or use of these "structural breaks," once identified, to more effectively apply various test-data filters, as is disclosed herein.

Data used to characterize behavior of nonlinear systems often includes discontinuities arising from sudden changes in those systems' excitation or response. In the context of material testing, load reversals or sudden changes in material or structural response due to plasticity, damage, etc. are examples of these sorts of sudden changes. In some cases, such as for brittle material failure, capturing the precise state at which such discontinuities occur can be a matter of primary interest.

Additionally, when dealing with signal data for a system to apply it to models representing that system (e.g., as in the case of exercising a material model with a strain/time history from a physical test and comparing the model's resulting stress/time history to the measured test response), it is commonly necessary to filter the signal data to reduce noise in the signal as well as reduce the overall frequency of the data points (e.g. reduce the number of data points for a given period of time). However, the accurate retention of these characteristic discontinuities in signals for systems' excitation and response is important to ensure that local temporal minima and maxima in addition to sudden changes in the system's excitation and response are accurately retained. This allows analysts, for example, to confirm and demonstrate that material models being considered exhibit the same characteristic excitation/response behavior as observed in the original signal. One objective of defining critical points is to provide a means for identifying important discontinuities in test data in a way that facilitates their retention when performing common test data filtering steps such as decimation, regularization, and smoothing which could otherwise result in the deletion or modification of these important characteristics of the test data.

In the systems and techniques disclosed herein, critical points can provide an analyst a means of persistently identifying history points in a collection of synchronized test data series that represent an important characteristic of the excitation or response, such as a load reversal, change in loading rate, sudden onset of nonlinear response, peak stress or force attained, or similar in the test data series. These points can be defined by a computer (e.g., through a simple user interface) and refined by toggling test data points as critical points, either by selecting them from a table or clicking the points on a graph to commit these points to the computer memory. Additionally, a straightforward algorithm is provided to identify excitation/response reversals to automatically define candidate critical points. More elaborate automated approaches could be applied to the framework in the future. In a typical implementation, the critical points identified persist with the test data in the computer memory, and once these critical points are defined, they cause other test data preprocessing tools (e.g., smoothing, regularization, and decimation) to modify the test data in a manner such that the critical points and the characteristic discontinuities they represent are retained without modification, thus preserving these important parts of the excitation/response history as the test data is modified.

Other technical differences may exist between various prior technologies and the systems and techniques disclosed herein.

### Technical Disclosure

Many products (e.g., tires, plastic cups, polymer membranes, etc.) deform when exposed to a load. The amount that a particular product will deform depends, at least in part, on the stiffness of the material that constitutes the part. A material model, in an exemplary implementation, is a virtual representation of an equation that describes the relationship between various material characteristics (e.g., applied force (or "stress") and resulting deformation (or "strain")) for a particular material. One exemplary type of material model is a linear elastic material model. There are many other types of material models.

In a typical implementation, a material model has one or more parameters. A parameter is essentially a numerical value in the material model that specifies or represents a property (e.g., stiffness) of a particular material. In a typical implementation, a material model can be calibrated to a particular material (e.g., steel, rubber, plastic, etc.) by assigning and/or adjusting the numerical values of the model's parameters so that the model characteristics match the observed characteristics of the actual material. Thus, calibrating a particular material model to different types of materials can require very different parameter values. For example, when deformation is small, both steel and rubber exhibit linear relationships between stress and strain and, therefore, may be reasonably represented by a linear elastic material model. If the linear elastic material model were calibrated to steel, for example, its parameters would be different than if the linear elastic material model were calibrated to rubber, and the material behavior represented by the steel-calibrated version of the linear material model would be different than the rubber-calibrated version of the same linear material model.

There are a variety of ways to calibrate a material model. Typically, calibration is performed based on a set of data that represents certain aspects of the material's behavior. For example, if a material model were to be calibrated to represent stress and strain behavior of a particular type of steel, then a selected material model might be calibrated based on test data that was collected from a real-world test of one or more specimens of the steel. The collected test data may include, for example, time-stamped stress and strain measurements taken by a real-world machine (e.g., a stress and strain test machine) while applying varying levels of stress to the test specimen(s) or subjecting the test specimens to various deformation modes or loading rates.

Very often, the raw data acquired from real-world machines used to measure material behavior is not well-suited for calibrating material models. Very commonly, such data includes far more data points than are required due to test machines outputting data at a very high rate, and this data often includes random perturbations about the actual measured characteristic quantity, typically called "noise". It is often sensible to process the test data set to modify it (e.g., reducing the number of data points in the test data set and/or filtering it to reduce the amount of noise present) before using the test data set to calibrate a material model. In many instances, this test data preprocessing can improve overall efficiency of the calibration process without meaningfully compromising accuracy of a material model calibrated using the preprocessed test data. In exemplary implementations, test data pre-processing can include, for example, decimation, regularization, and/or smoothing of the test data. In a typical implementation, such preprocessing of the test data set results in a modified version of the test data set that has fewer and/or different test data points than the test data set had before any preprocessing occurred.

To ensure a high degree of usefulness in the test data set after preprocessing and a high degree of accuracy in a material model calibrated using the preprocessed test data set, it is important that certain, more critical, test data points remain intact and not be compromised, modified, or removed from the test data set during preprocessing. In a typical implementation, the systems and techniques disclosed herein facilitate automatically identifying and keeping intact such critical test data points (or critical points) throughout any test data preprocessing, so that those critical test data points are weighed into any subsequent material model calibration. In a typical implementation, the systems and techniques disclosed herein enable a human user's ability to manually designate any critical test data points (i.e., ones to be preserved in the test data set throughout preprocessing) as an alternative to, or in addition to, any automatic identification (by a computer) of critical test data points.

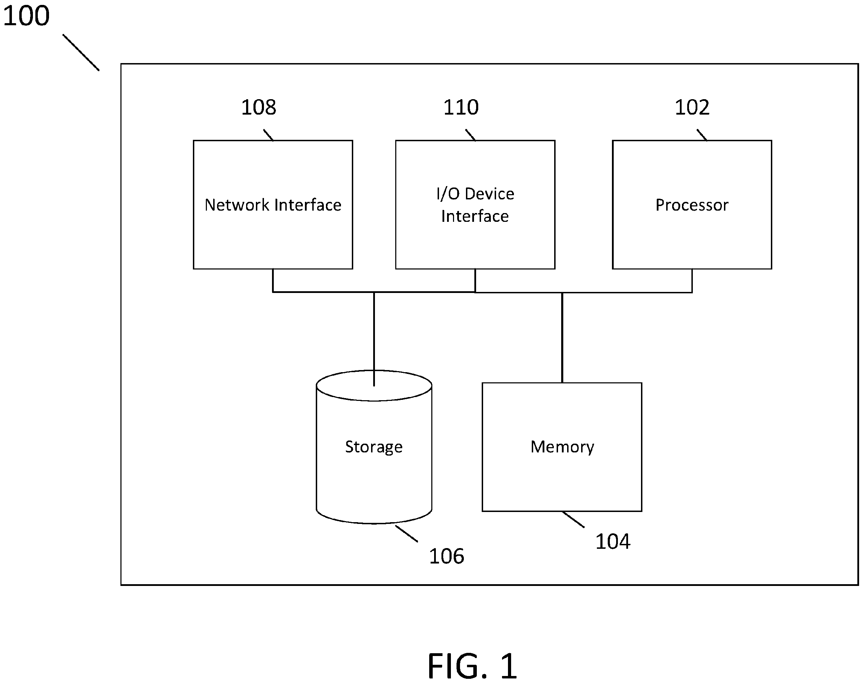

FIG. 1 is a schematic representation of an exemplary computer 100 that is configured to generate a highly accurate calibrated material model based on a set of data points and utilize that highly accurate calibrated material model to analyze a design that includes the modeled material. More specifically, in a typical implementation, the system 100 is configured to preserve critical data points from the data point set throughout any test data preprocessing that may be applied to the test data set before it is used to calibrate the material model.

The illustrated computer 100 has a processor 102, computer-based memory 104, computer-based storage 106, a network interface 108, an input/output device interface 110, and a bus that serves as an interconnect between the components of the computer 100. The bus acts as a communication medium over which the various components of the computer 100 can communicate and interact with one another.

The processor 102 is configured to perform the various computer-based functionalities disclosed herein as well as any other supporting functionalities not explicitly disclosed herein. Some of the computer-based functionalities that the processor 102 performs include generating a highly accurate calibrated material model based on a set of data points and/or utilizing the highly accurate calibrated material model to analyze a design that includes the modelled material. Moreover, in some implementations, the processor 102 is configured to preserve critical data points from the data point set throughout any test data preprocessing that may be applied to the test data set before it is used to calibrate the material model. Typically, the processor 102 performs these and other functionalities by executing computer-readable instructions stored on a computer-readable medium (e.g., memory 104 and/or storage 106). In various implementations, some of the processor functionalities may be performed with reference to data stored in one or more of these computer-readable media and/or received from some external source (e.g., from an I/O device through the I/O device interface 110 and/or from an external network via the network interface 108). In an exemplary implementation, the processor is embodied in one or more hardware devices that are configured to perform computer-based processing functionalities, by executing computer-readable instructions stored, for example, in computer-based memory such as, for example, storage 104 or memory 106 in FIG. 1.

The computer 100 in a typical implementation has volatile and non-volatile memory / storage capabilities. More specifically, in a typical implementation, memory 104 provides a form of volatile storage for computer-readable instructions that, when executed by the processor 102, cause the processor 102 to perform or facilitate some (or all) of the computer-based functionalities disclosed herein. Moreover, in a typical implementation, storage 106 provides a form of non-volatile memory storing computer-readable instructions, such as instructions to implement an operating system, configuration information, etc. that, when executed by the processor 102, cause the processor 102 to perform some (or all) of the computer-based functionalities disclosed herein. Each of these system memory resources (e.g., 104, 106) may store data, as well.

In a typical implementation, the memory 104 stores computer-readable instructions that, when executed by the processor 102, cause the processor 102 to execute functionalities that present to a user at the computer 100 a computer-based simulation program, for example, with the functionalities disclosed herein incorporated and/or available for access to a human user therein. An example of a computer-based simulation program is available on the computer-based 3DEXPERIENCE^{®} platform, available from Dassault Systèmes SE. The computer-based simulation program, so adapted, would include the material model calibration and other functionalities disclosed herein.

The network interface 108 is a component that enables the computer 100 to connect to any one or more of a variety of external computer-based communications networks, including, for example, a local area network (LANs), a wide area network (WANs) such as the Internet, or the like. In various implementations, the network interface 108 can be implemented in hardware, software, or a combination of hardware and software.

The input/output (I/O) device interface 110 is a component that enables the computer 100 to interface with any one or more input or output devices, such as a keyboard, mouse, display, microphone, speakers, printers, etc. In various implementations, the I/O device interface can be implemented in hardware, software, or a combination of hardware and software. In a typical implementation, the computer may include one or more I/O devices (*e.g*., a computer screen, keyboard, mouse, printer, touch screen device, etc.) interfaced to the computer 100 via 110. These I/O devices (not shown in FIG. 1) act as human-machine-interfaces (HMIs) and are generally configured enable a human user to interact with the system 100 to access and utilize the functionalities disclosed herein.

In an exemplary implementation, the computer 100 is connected to a display device (e.g., via the I/O device interface 110) and configured to present at the display device a visual representation of an interface to a product design and/or simulation environment, such as the one available through the aforementioned 3DEXPERIENCE^{®} platform. The interface and its visual representation on the computer-based display device, in a typical implementation, provides the user with access to the functionalities disclosed herein, and displays (e.g., on a display device coupled to the I/O device interface 110) a visual representation of information related to those functionalities.

In some implementations, the computer 100 and its various components may be contained in a single housing (e.g., as in a personal laptop) or at a single workstation. In some implementations, the computer 100 and its various components may be distributed across multiple housings, perhaps in multiple locations on a network. Each component of the computer 100 may include multiple versions of that component, possibly working in concert, and those multiple versions may be in different physical locations and connected via a network. For example, the processor 102 in FIG. 1 may be formed from multiple discrete processors in different physical locations working together to perform processes attributable to the processor 102 as described herein, in a coordinated manner. A wide variety of possibilities regarding specific physical configurations are possible.

In various implementations, the computer 100 may have additional elements not shown in FIG. 1. These can include, for example, controllers, buffers (caches), drivers, repeaters, receivers, etc. The interfaces (e.g., 108, 110) in particular may include elements not specifically represented in FIG. 1, including, for example, address, control, and/or data connections to facilitate communications between the illustrated computer components.

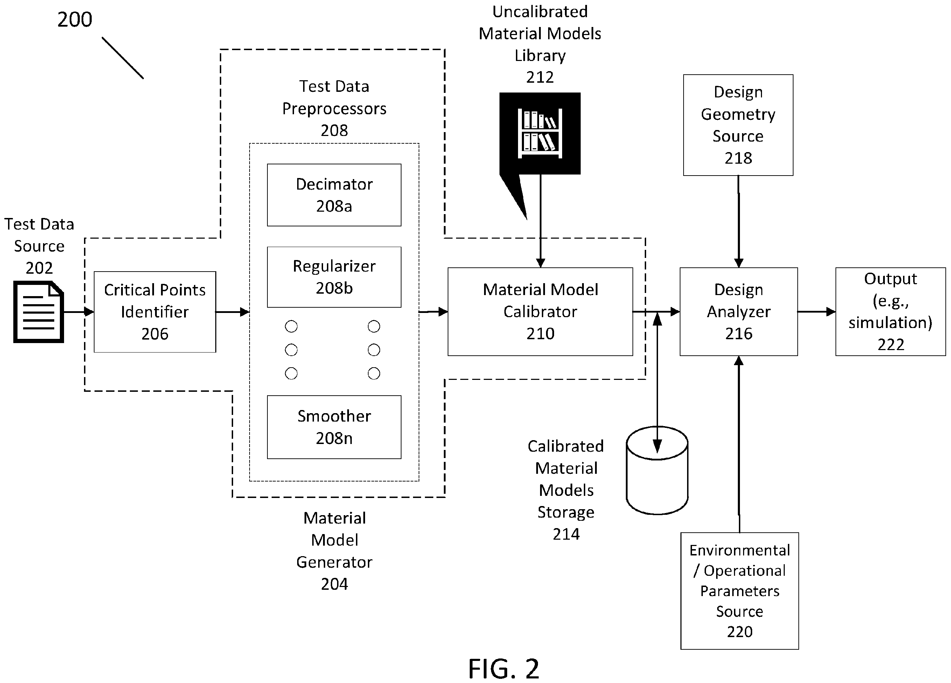

FIG. 2 is a schematic representation of an exemplary system 200 for generating a highly accurate calibrated material model and utilizing that calibrated material model to analyze a design that includes the modelled material. More specifically, the system 200 is configured to preserve any critical test data points throughout any test data preprocessing that is applied to the test data set before it is used to calibrate the material model. In one exemplary implementation, part (or all) of the system 200 in FIG. 2 may be implemented or embodied on the computer 100 in FIG. 1.

The illustrated system 200 has a source of test data 202, a material model generator 204, a library of uncalibrated material models 212, storage for calibrated material models 214, a design analyzer 216, a source of design geometry 218, a source of environmental / operational parameters 220, and an output 222. The material model generator 204 in the illustrated implementation has a critical points identifier 206, test data preprocessors 208 (including, in the illustrated example, a decimator 208a, a regularizer 208b, and a smoother 208n), and a material model calibrator 210. The various components are interconnected, at least as shown, to facilitate communication and exchange of data therebetween. In various implementations, the material model generator 204 may be integrated into a Material Calibration Application, such as the Material Calibration application in the 3DEXPERIENCE^{®} platform.

The source of test data 202 can be any one of a variety of computer-based resources for storing digital data in a computer storage device. Some examples of possible test data sources 202 include a Microsoft Excel^{®} spreadsheet, a comma separated values (CSV) file, and/or an ASCII file stored in computer-based storage (e.g., 106).

The test data itself can include a wide variety of different types and amounts of test data. In an exemplary implementation, the test data comprises a series of time-stamped, test data points measured by a real-world test instrument during a real-world test of a particular material specimen. For example, in some implementations, the test data includes, in tabular form, a listing of time stamped stress and strain measurements taken during a real-world stress test applied to a rubber specimen using a stress and strain machine with varying levels of stress being applied to the specimen over time.

The test data may be stored in any number of ways including, for example, in a tabular format or any other convenient format.

The material model generator 204 can be virtually any kind of computer-based component or components configured to perform the functionalities disclosed herein associated with generating a calibrated material model. More specifically, in the illustrated implementation, the material model generator 204 generates the calibrated material model from an uncalibrated material model using test data obtained from the test data source 202. Moreover, in a typical implementation, the material model generator 204 facilitates various preprocessing options for the test data but preserves any critical test data points throughout any test data preprocessing that gets applied to the test data before using it for calibration purposes.

In various implementations, the material model generator 204 (and/or any one or more of its subcomponents 206, 208, 210) may be embodied by one or more processors (e.g., 102 in FIG. 1) executing computer-readable instructions stored in a computer-readable medium (e.g., 104 and/or 106 in FIG. 1) that cause the processor 102 to execute computer functionalities described herein as being attributable to the material model generator 204 and/or its subcomponents 206, 208, 210.

Referring again to FIG. 2, the illustrated system 200 has a library of uncalibrated material models 212 that the material model generator 204 can access for calibrating. In a typical implementation, the library of uncalibrated material models 212 is stored in a computer-readable medium (e.g., storage 106 in FIG. 1). In various implementations, the library of uncalibrated material models 212 may contain any number of different types of material models. These material models may be used to model many materials include, for example, metals, rubber, concrete, etc. The material models available in the library of uncalibrated material models can include, for example, one or more elasticity models, plasticity models, hyperelasticity models, viscoelasticity models, creep models, Nitinol models, models for user-defined materials, etc.

The system 200 also has storage for calibrated versions of the material models 214. In a typical implementation, the calibrated material models storage 214 is embodied in a computer-readable medium (e.g., storage 106 in FIG. 1). The calibrated material models storage 214 is configured to and does store calibrated versions of material models that are generated, for example, by the material model generator 204.

The system 200 has a design analyzer 216. The design analyzer 216 can be virtually any kind of computer-based component or components configured to perform the functionalities disclosed herein or otherwise and associated with design analyses. Examples of design analyses may include simulating a process utilizing finite element analysis involving one or more aspects of a computer-based design. In the illustrated example, the design analyzer 216 is configured to receive, as inputs, a material model calibrated to a particular material, design geometry for a product that includes the particular material, and/or environmental and/or operational parameters.

The calibrated material model in the illustrated system comes to the design analyzer 216 from the material model calibrator 210 or the calibrated material models storage 214. The design geometry in the illustrated system is provided to the design analyzer 216 from a design geometry source 218. The design geometry source 218 can be a computer-aided design (CAD) program, such as the Solidworks^{®} program, executing on computer 100. The design geometry in this example, may be provided from the CAD program to the design analyzer 216 in the form of one or more CAD drawings. The environmental and/or operational parameters in the illustrated system is provided to the design analyzer 216 from a source 220 of those parameters. The source 220 of the environmental and/or operational parameters can be one or more input/output (I/O) devices (connected, e.g., to 110 in FIG. 1) and may be entered by a human user through an interface that prompts or enables the human user to enter or specify the parameters. The environmental or operational parameters can include, for example, temperatures, pressures, speeds, settings, etc.

In an exemplary implementation, the design analyzer 216 is configured to conduct simulations of a design based on a design geometry made from a particular material, a material model for the particular material and user-specified parameters for the simulation. The design analyzer 216 produces an output 222 from its simulation, for example, on a display screen (of computer 100), etc. In a typical implementation, the enhanced calibrated material models produced by the system 200 result in enhanced simulation results (at 222), improved understanding of a design and improved ability to optimize the design for particular objectives.

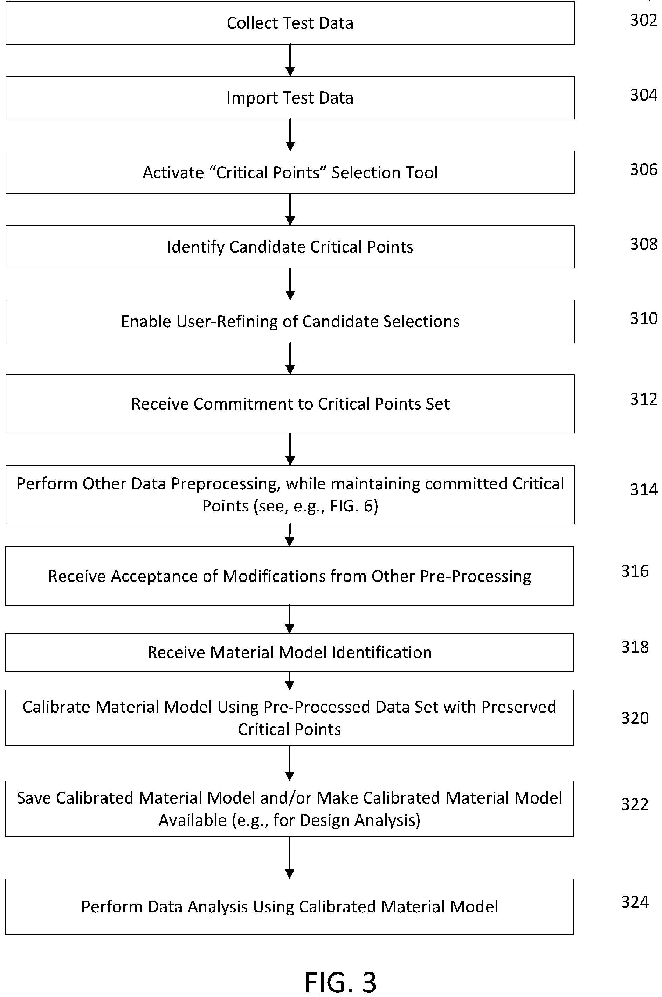

FIG. 3 is a flowchart that represents an implementation of a process for producing a calibrated material model that may be used, for example, for design analysis.

The process represented in the illustrated flowchart begins with collecting test data (at 302). This step (302) may be performed in any number of ways. In an exemplary implementation, the test data may be obtained (at 302) by using real-world physical machinery (e.g., a stress and strain test machine) to apply a load (compression or tension) to a specimen of material (e.g., rubber, plastic, steel, etc.) and measure the resulting stress and strain at different times during the test. In some implementations, the loading may be varied (e.g., increased and decreased, perhaps cyclically) throughout the test. In an exemplary implementation, strain may be measured using a strain gauge, which may be integrated into the test machine, for example, and stress may be derived from the force measurements and known properties (specifically the dimensions and, more specifically, the cross-sectional area) of the material specimen being tested. The test results may include time-stamped stress and resulting strain values. These test results are loaded as digital data and stored in a computer-readable medium (e.g., in 104 in FIG. 1, as an Excel^{®} spreadsheet). In various implementations, the test results can be loaded automatically by the test machine itself or by a human observer manually entering the test results. The resulting storage file (e.g., Excel^{®} spreadsheet) represents a source of test data (202) representing real-world, experimental test data for the tested type of material.

Next, in illustrated implementation, (at 304) the test data is imported from the test data source 202 to the material model generator 204. In one specific implementation, in this step (304) the experimental test data is loaded into a computer-based material calibration tool. An example of an existing computer-based material calibration tool is the Material Calibration application which is part of the 3DEXPERIENCE^{®} platform, available from Dassault Systèmes SE. The computer-based material calibration tool in this regard may enable the human user to import the test data from a source file (e.g., an Excel^{®} spreadsheet) into the tool for use in connection with producing a calibrated material model for the corresponding tested material. In this regard, the computer-based material calibration tool may present to the human user (at a display screen of computer 100) an "import test data" button, the selection of which opens a dialogue that enables the human user to specify the location in memory 104 where the source of test data 202 resides, then, in response to the user identifying the test data source 202, automatically importing the test data into the computer-based material calibration tool for use (e.g., by material model generator 204) in producing a calibrated material model.

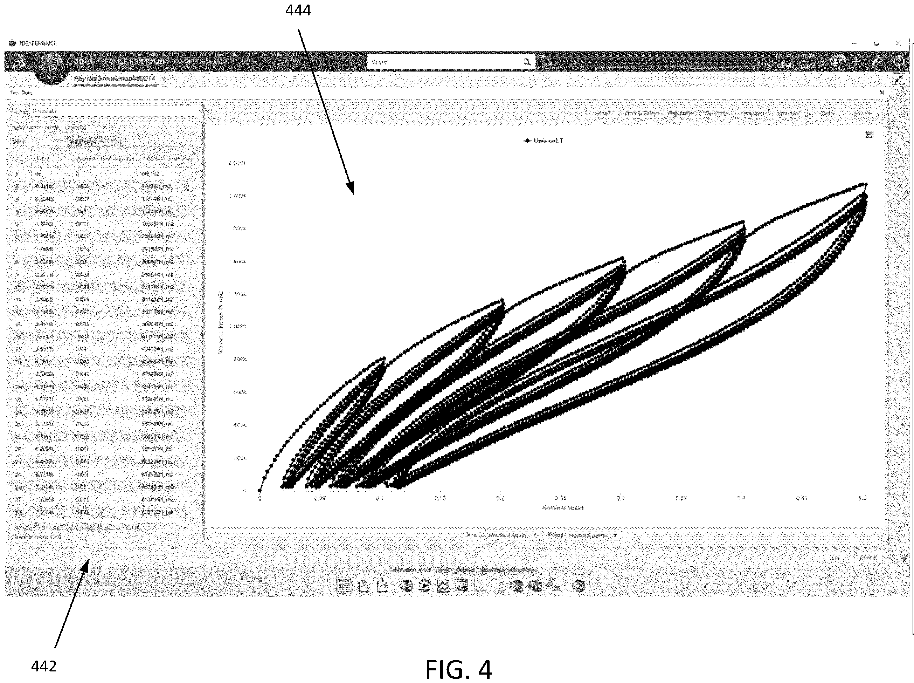

FIG. 4 shows a screenshot from a version of 3DEXPERIENCE^{®} Material Calibration application, adapted according to the systems and techniques disclosed herein. The screenshot includes a table 442 (at the left side of the screenshot) and a graph 444 (at the right of the table) with an exemplary set of cyclic time-based stress-strain test data. In this test, a material specimen was initially loaded up to some strain, then cycled between that strain level and a strain yielding zero stress several times before being loaded up to a higher strain level. At each subsequent strain loading, the specimen is cycled in this manner multiple times, resulting in many cycles and C1 discontinuities in the stress and strain time histories. The table 442 includes a listing of time, nominal uniaxial strain, and nominal uniaxial stress for each measurement taken during the test. The table 442 may be populated directly from the test data source (e.g., Excel^{®} spreadsheet). The graph 444 has an abscissa (x-axis) that represents unitless nominal strain and an ordinate (y-axis) that represents nominal stress (in N.m²). The plot in the graph 444 represents the data in the table 442. Next, in the illustrated example, the computer 100 (at 306) receives a prompt to activate the critical points identifier 206. In an exemplary implementation, the prompt may come in response to a human user taking some action at the computer 100 (e.g., by selecting a "Critical Points" option 445 presented to the user within the Material Calibration application). Once the "Critical Points" option has been selected, the user may then select the "Evaluate" button 447 to cause the critical points identifier 206 to automatically identify candidate critical points in the imported test data.

The computer 100 then (at 308) automatically identifies candidate critical points among the imported test data. The word "automatically" here refers to the fact that after the user initiates the critical point identification process, the identification process (308) is performed by the computer 100 (e.g., the computer processor 102) without requiring further input or involvement by the user. There are a variety of ways in which the computer 100 may perform this identification process (308).

In one exemplary implementation, the critical points identifier 206 automatically identifies any points in the imported test data that represent a reversal in a data series within the imported test data and flags those points as candidate critical points.

In various implementations, the critical points identifier 206 may identify these reversal-type candidate critical points (at 308) by identifying any data points for which the immediately preceding data point and the immediately following data point are both greater than or both less than the current point (a true "reversal"). In various implementations, the critical points identifier 206 may identify candidate critical points (at 308) by identifying any data points for which one of the immediately preceding data point or the immediately following data point is equal and the other of the immediately preceding data point or the immediately following data point is not equal. This indicates a point in the series where the measurements are transitioning from a constant value to a different and possibly changing value. In some implementations, the critical points identifier 206 identifies candidate critical points (at 308) by calculating best-fit lines for some number of test data points preceding and following a particular test data point and identifying the particular test data points for which the magnitude of change in slope between the preceding and following best fit lines reaches a local maximum. Other methods are possible for identifying candidate critical points (at 308). In some implementations, the critical points identifier 206 implements more than one of (or all) these approaches for identifying candidate critical points (at 308).

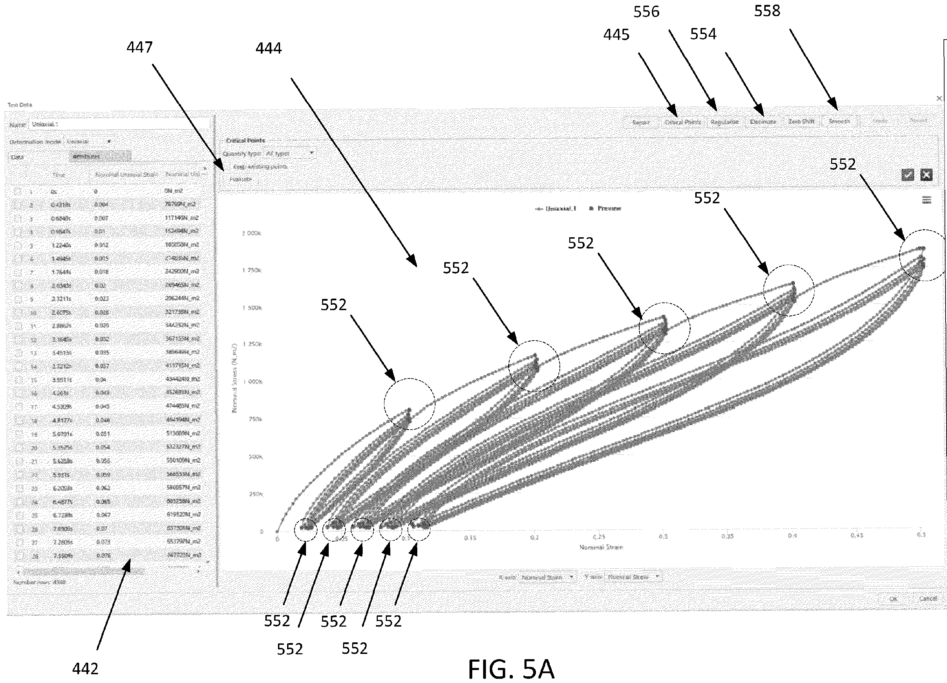

In an exemplary implementation, once the critical points identifier 206 identifies its candidate critical points (at 308), the candidate critical points so identified may be visually denoted on the computer's 100 display. FIG. 5A is a screenshot that is like the screenshot in FIG. 4, except that the FIG. 5A screenshot shows an example of how the critical points identified by the critical points identifier 206 may be visually denoted on the computer's 100 display.

More specifically, in the exemplary screenshot of FIG. 5A, the plot of data points in the tool's plot panel 444 uses visually distinct markers to indicate each critical point identified by the critical points identifier 206. The visually distinct markers can take on any number of possible forms and act to visually distinguish the data points identified as being critical from the data points not identified as being critical. In the illustrated example, the visually distinct markers are overlaid on the data points that have been identified as being critical. The visually distinct markers may have a different color, size, and/or shape than the visual indicators used by the computer 100 to identify data points that were not identified as critical points. In the specific example shown, dashed circles 552 (which would not necessarily appear in the screenshot) identify where in the graph clusters of critical points have been identified. More specifically, within each circle 552, there are multiple dots that are visually more prominent than other dots in the graph. Each of these visually more prominent dots is a data point that has been identified as a critical point by the critical points identifier 206.

Additionally, the listing of test data points in table 442 includes a box next to (and to the left of) each entry. In an exemplary implementation, the computer 100 would populate these boxes to distinguish data entries that correspond to critical points from data entries that correspond to non-critical points. In some implementations, the computer 100 would populate the boxes that correspond to critical data points with a check mark or an "x" mark for example and leave the other boxes that correspond to non-critical data points empty. The data entry for any checked or "x" marked boxes in table 442 would correspond to a point visually denoted as being critical in plot panel 444.

Referring again to FIG. 3, the computer 100 (at 310) enables the user to refine the critical point selections that were automatically made by the critical points identifier 206 (at 308).

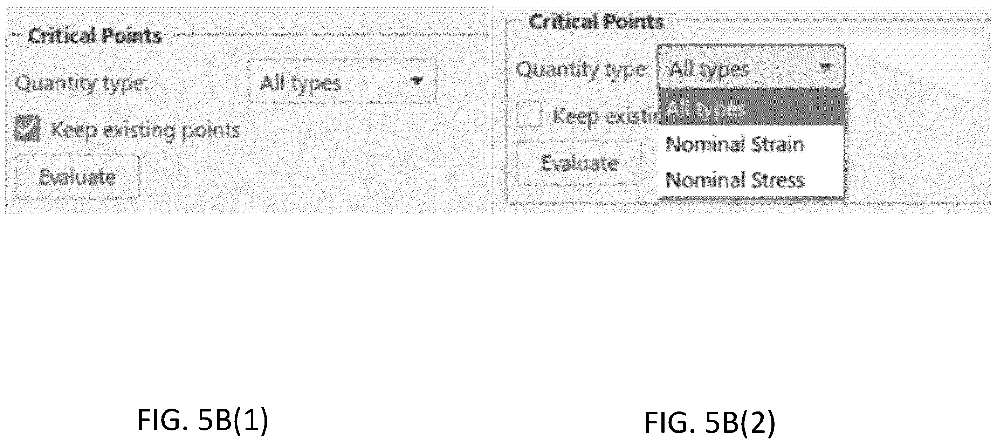

For example, the computer 100 typically gives the user the option to change the selection process to only include one of the data series (e.g., to only include reversals in the strain or in the stress, but not both). The user also has the ability to keep previously-defined critical points, allowing the user to quickly build up critical points based on certain series. In this regard, the computer 100 may make available to the user (e.g., at the computer display), an interface element like the one shown in FIG. 5B(1) and 5B(2).

In addition to this automatic tool for specifying candidate critical points, the user can directly toggle the check boxes in the table of test data (in FIG. 5A) to change which data points will or will not be flagged as critical points.

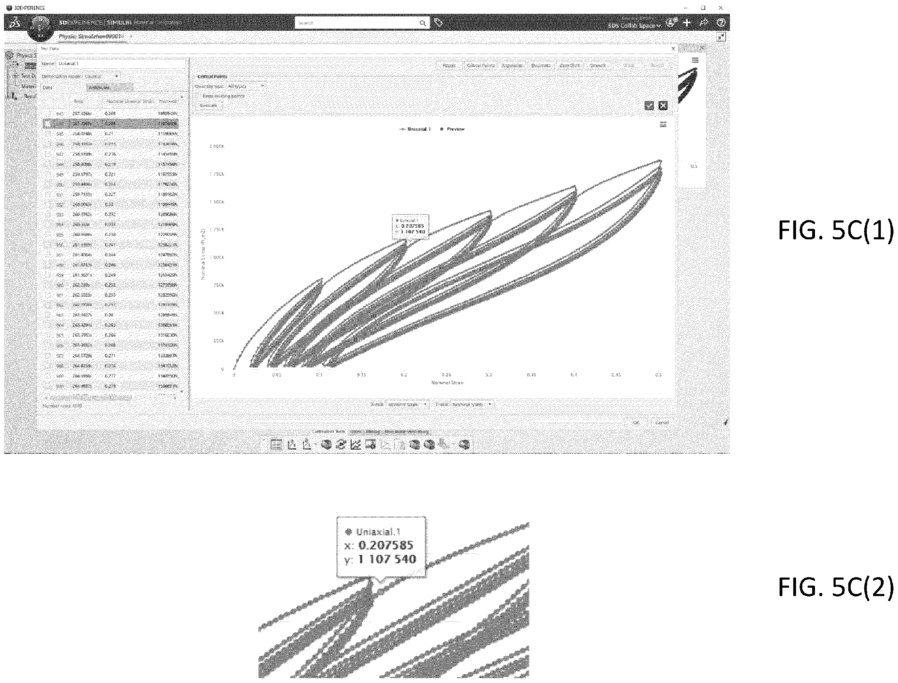

Additionally, the user can directly click on data points in the plot to highlight the corresponding entry in the table where the checkbox can be toggled to facilitate quick visual-based specification of critical points. (*See, e.g*., FIG. 5C(1) and 5C(2)).

Once the user has completed flagging the candidate critical points, he or she can click the checkmark button in the upper right-hand corner of the screen to commit the selection to the dataset. Each critical point is then flagged in the table with an asterisk (*) next to its index in the table. Additionally, red (or other visually prominent) markers are overlaid on the plot of the test data to identify the critical points.

Refinement (at 310) may involve, for example, the user manually designating one or more additional data points as critical. This may make sense if, for example, the user wants to ensure that those one or more additional data points are preserved in the test data set through subsequent data preprocessing for use in material model calibration.

Referring again to FIG. 3, next (at 312), the computer 100 receives a commitment to the (refined or not) critical points set. In a typical implementation, this step may involve a user entering an indication at the computer 100 that the critical points selected by the critical points identifier 206 (and optionally refined by the user at 310) are acceptable and that the resulting data set (with the critical points) is ready for any additional data preprocessing (including, for example, decimating, regularizing, and/or smoothing) that may be desired prior to using the data to calibrate a material model.

Next (at 314), according to the illustrated process, the material model generator 204 performs additional data preprocessing as desired. Examples of additional data preprocessing include decimating, regularizing, and/or smoothing the data. Referring to the screenshot of FIG. 5A, for example, to initiate decimating (at 314), the user may select the decimate button 554 on the screenshot of FIG. 5A. This selection causes the decimator 208a in the test data preprocessors 208 to decimate the test data set as disclosed herein. To initiate regularizing (at 314), for example, the user may select the regularize button 556 on the screenshot of FIG. 5A. This selection causes the regularizer 208b in the test data preprocessors 208 to regularize the test data set as disclosed herein. To initiate smoothing, for example, the user may select the smooth button 558 on the screenshot of FIG. 5A. This selection causes the smoother 208n in the test data preprocessors 208 to smooth the test data as disclosed herein.

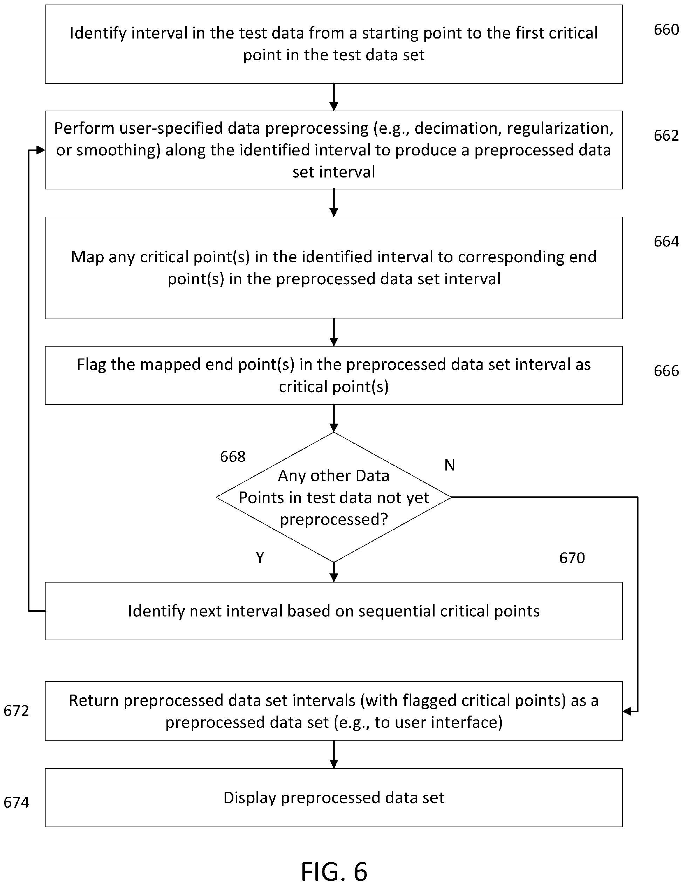

FIG. 6 is a flowchart that represents an exemplary implementation of test data preprocessing in a manner that preserves any of the critical points identified by the critical points identifier 206 or the user.

First, according to the illustrated flowchart, the computer 100 (at 660) identifies an interval in the test data (e.g., from a first data point (which may itself be a critical point) in the test data set to, and optionally including, the first critical point in the test data set after the first data point). The computer 100 can do this because the critical points in the test data set already were identified / flagged in 308 to 312. In some implementations, the computer 100 creates this interval by starting from the first data point in the test data set and checking each data point in the test data set, sequentially, for a critical point flag (e.g., a representation in computer memory that the data point has been flagged as critical). Every data point that the computer 100 identifies as not having a critical point flag and the first data point in the sequence that has a critical point flag get added to the interval. The interval is considered completed once the computer reaches that first data point flagged as critical and adds that critical data point to the interval. The resulting test data interval includes all the data points up to and including the first critical point in the sequence. The resulting test data interval is made available (e.g., to the decimator, regularizer, or smoother, as appropriate) to be preprocessed according to the user's preprocessing desires, as indicated by the user's prior selection.

Next, according to the illustrated flowchart, (at 662) a corresponding one of the test data preprocessors 208 performs the corresponding data preprocessing (e.g., decimating, regularizing, or smoothing), according to the user's prior selection, along the identified interval to produce a preprocessed data set interval. Because the only critical point(s) in the interval are at the end of the interval, the critical points are preserved during the data preprocessing, regardless of whether the data preprocessing involves decimation, regularization, or smoothing. Moreover, because none of the other data points in the interval are critical points, no critical points are lost during the data preprocessing.

Next, according to the illustrated flowchart, (at 664), the computer 100 maps the critical point that is at the end of the identified test data interval to the last data point in the preprocessed data set interval. Mapping can be implemented in any one of a number of possible ways. Typically, mapping includes creating a logical association in computer memory 104 between the two values (i.e., between the critical point at the end of the identified test data interval and the last data point in the preprocessed data set interval). Mapping facilitates effective flagging of critical points in the preprocessed data set.

In cases where preprocessing rewrites a test data series (for instance, in regularization which resamples the test data), the critical point(s) in the original test data series are mapped to equivalent critical points in the new test data. For instance, consider a test data object (containing a test data set) for which the 1458^{th} point is flagged as the first critical point. Suppose this test data object is then regularized so that there are only 100 data points before the 1^{st} critical point. In the modified test data object, the first 100 points would be resampled from the original data with equal interval spacing based on time or frequency, and the 101^{st} point would be mapped to the 1458^{th} point (i.e., the first critical point) in the original test data object and would be flagged, therefore, as the first critical point in the modified test data object. Then, for all the data series (e.g., time, strain, stress, etc.) the values of the 101^{st} entry would be equal to, and mapped to, and flagged as critical like the 1458^{th} entry of the original test data set.

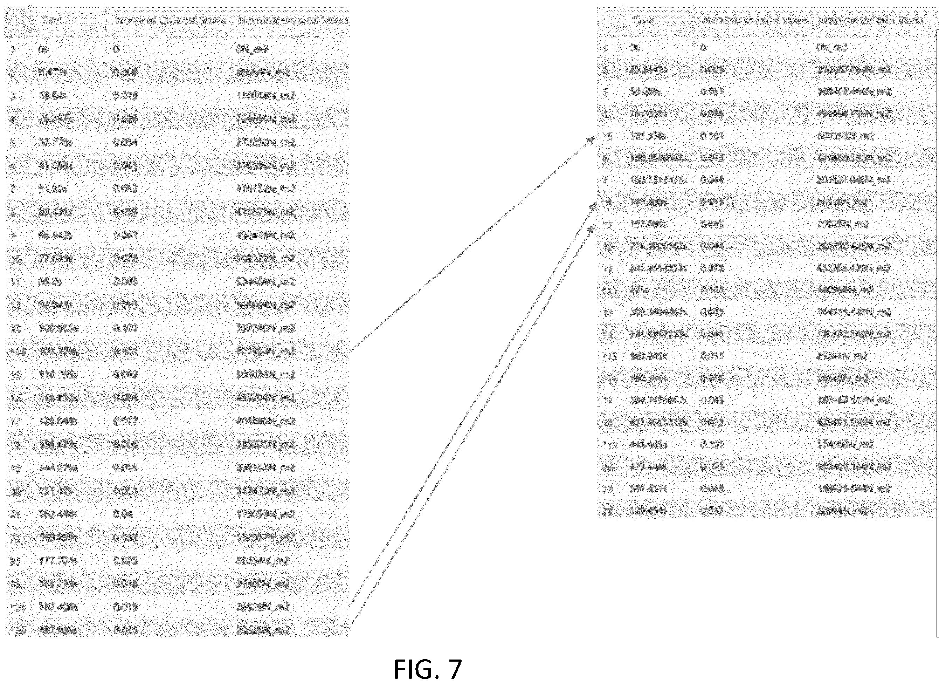

FIG. 7 shows an example of test data that gets regularized, noting the mapping (a logical relationship represented, e.g., in computer memory 104) between critical points in the test data object before and after regularization. In this example, only the first 26 test data points in the original test data object are shown in FIG 7, but this test data object contains more test data points than are shown. The mapping for critical points is 1:1, i.e., every critical point in the original data maps to the equivalent critical point in the regularized data.

Referring again to the flowchart of FIG. 6, next (at 666), the computer 100 flags the last data point in the preprocessed data set interval (that has been mapped to the critical point from the original data set) as a critical point. Setting this flag typically involves creating a designation in computer memory (e.g., 104) to indicate that the associated data point is a critical point and should be treated as such. The computer 100, in a typical implementation, would be configured to display such a flagged data point with appropriate prominence in a visual display of the preprocessed data (e.g., at the computer's display screen).

Next, according to the illustrated flowchart, (at 668), the computer 100 may consider whether there are any other data points in the original test data set that have not yet been preprocessed (e.g., decimated, regularized, or smoothed). There are a variety of ways that this step may be performed. In one example, the computer 100 is configured to maintain a pointer in memory pointing to the last data point preprocessed. In those instances, the computer 100 may pick up preprocessing from wherever the pointer is pointing.

If the computer 100 (at 668) determines that there are other data points in the original test data set that have not yet undergone preprocessing, then the computer 100 (at 670) identifies the next interval of data points for preprocessing. In a typical implementation, the next interval would extend from the last critical point at the end of the prior interval to the next critical point in the original test data series. However, if the end of the test data series is being approached, then the next interval would extend from the last critical point at the end of the prior interval to the last data point in the original test data sequence.

Continuing the example above, after the first interval of data points (from the first data point to the first critical point) has been preprocessed (at 662), the next interval of data points (identified at 670) might extend from (and optionally include) the first critical point to (and optionally including) the next critical point in the original test data series. Since, in some implementations, any critical points may fall at the beginning or end of each interval, applying the preprocessing functionalities (at 662) across a particular interval will naturally preserve the end points (i.e., the critical points). Moreover (at 664), the critical points that define any subsequent intervals will be mapped to corresponding points (e.g., at the beginning or end of resulting preprocessed intervals) and flagged accordingly (at 666). It is worth noting that, in various implementations, an interval does not necessarily contain a critical point, but it usually does. Any critical points will always be at the beginning and/or end of an interval.

It is also worth noting that, in a typical implementation, the preprocessing functions, at their core, contain kernel functions that are designed to operate on an interval of data while preserving endpoints. For the smoothing functionality, for example, it assumes a certain degree of continuity within the interval that would tend to smooth out sudden slope changes in the interval. By treating the data on either side of the critical point as separate intervals, the discontinuity at the critical point is preserved.

In general, a first one of the intervals can be defined by an initial point in the first test data set and a first one of the critical points, a second one of the intervals can be defined by a last one of the critical points and an end point in the first test data set, and other ones of the intervals can be defined by sequential critical points in the first test data set. Critical points can be considered sequential in this regard as long as there are no other critical points between them. Non-critical data points can be present between sequential critical points.

If the computer 100 (at 668) determines that there are no other data points in the original test data set that have not yet undergone preprocessing, then the computer 100 (at 672) returns the preprocessed data set intervals. In a typical implementation, these intervals might be returned to a user interface for display (at 674) as a plot or in tabular form, in correct sequential order, for example, with critical points visually identified, on a computer display. It is worth noting that, in various implementations, the last data point in a given data set may or may not have been flagged as a critical data point. Regardless, in a typical implementation, the last data point in the test data set may be treated as if it were a critical data point.

Referring again to FIG. 3, after the preprocessing is performed (at 314) and the results are displayed to the user at the computer 100, the user can review those results and accept them. In some implementations, the computer 100 enables the user to modify the results produced by the preprocessing (at 314) in much the same way that the user was able to refine candidate selections at 310. In a typical implementation, after preprocessing (at 314), the computer 100 also provides the user with a way to accept the preprocessing results as provided by the computer 100 and/or as modified by the user. For example, the computer 100 may present at the display a user-selectable "accept" or "commit" button, the selection of which causes the data set (after preprocessing and/or other user modifications) to be committed toward use in material model calibration. The exemplary process represented in the illustrated flowchart shows the computer 100 (at 316) receiving just such an indication of acceptance or commitment.

In a typical implementation, the computer 100 enables a user to select a particular material model for calibration using the preprocessed data with preserved critical points (accepted at 316). In some implementations, the computer 100 in this regard stores a library (i.e., a data collection) of uncalibrated material models (see, e.g., 212 in FIG. 2). The computer 100, in a typical implementation, presents a user-selectable listing of uncalibrated material models available in the library on the computer's display. The user can view the available options and select one of the uncalibrated material models for calibration using the preprocessed data (accepted at 316). The illustrated flowchart shows the computer 100 receiving a user selection (at 318) identifying a particular one of the uncalibrated material models for calibration from the library 212.

Next (at 320), the computer 100 calibrates the user-selected material model using the preprocessed data (with preserved critical points) that was accepted at 316. In various implementations, the tool may allow the user to select a subset of imported test data objects (preprocessed or not) to perform the calibration. In other words, for example, the user may have input 10 test data sets but chooses to only use one of them to calibrate the model.

Material model calibration involves defining and/or adjusting material parameters represented in the user-selected uncalibrated material model based on the preprocessed data that was accepted at 316 in order to adjust how the material model operates and might simulate real world processes involving the material. In a typical implementation, the material model will be calibrated so as to predict material behaviors that closely match the behavior represented by the preprocessed data used to calibrate the material model. The specifics of material model calibration can vary. However, there are a variety of existing applications that can perform and/or facilitate material model calibration. One example is the material model calibration application available on the 3DEXPERIENCE^{®} platform, available from Dassault Systèmes SE. Another example is the MCalibration^{®} tool from Veryst Engineering, LLC. Either of these, or any other material model calibration systems or processes can be implemented or utilized to calibrate the user-selected material model (at 320) to produce a calibrated version of the material model.

After calibration (at 320), according to the illustrated flowchart, the computer 100 (at 322) saves the calibrated material model (e.g., in 104 of FIG. 1) and/or makes it available for further analysis.

Next, according to the illustrated flowchart, the computer 100 (at 324) utilizes the calibrated material model to analyze a design that includes the associated material. In an exemplary implementation, this design analysis (at 324) takes into account design geometry from source 218 and/or environmental / operational parameters 220 and produces an output 222 (e.g., on the computer display) that may result or involved a transformation or evolution of the design geometry, for example. In a typical implementation, the inclusion (and preservation) of critical points in the material model calibration processes disclosed herein, makes any design analysis that involves the calibrated material model more accurate and effective than it otherwise might be.

One purpose of the preprocessing - that is performed, e.g., at 314 - is to convert test data from something that is generally acquired in a test lab which may contain noise and is often sampled at very high frequency and convert it into data which is suitable for performing evaluations of numerical material models for the purpose of optimization (calibration). As discussed, the preprocessing functionalities disclosed herein preserve any critical points. The three pre-processing algorithms specifically mentioned herein, which interact with the critical point functionality are data decimation, data regularization, and data smoothing. The overriding principle which these pre-processing tools follow is that critical point data should be preserved unchanged when modifying/filtering data.

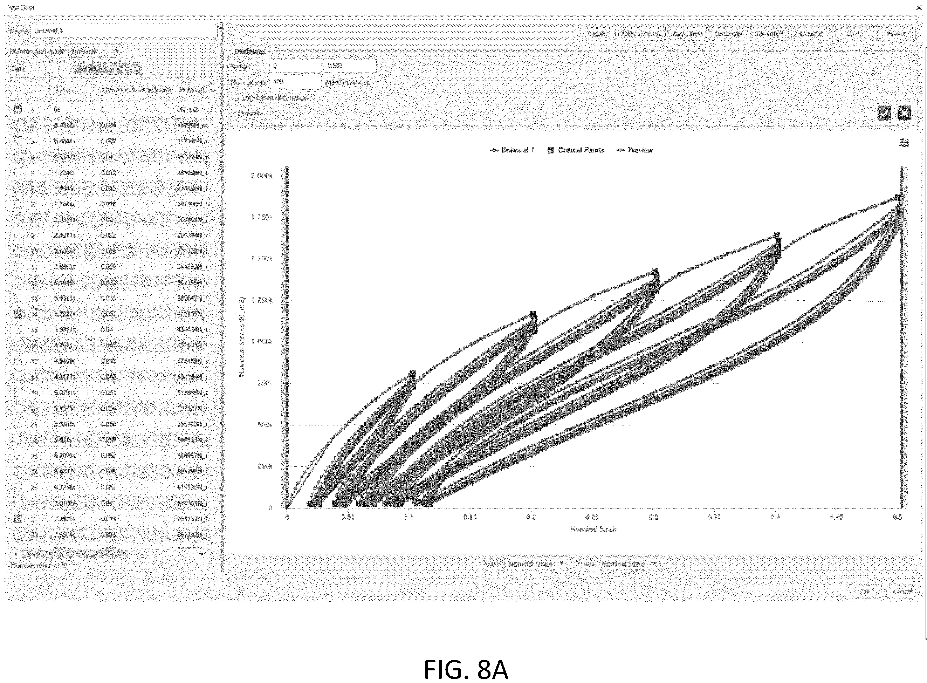

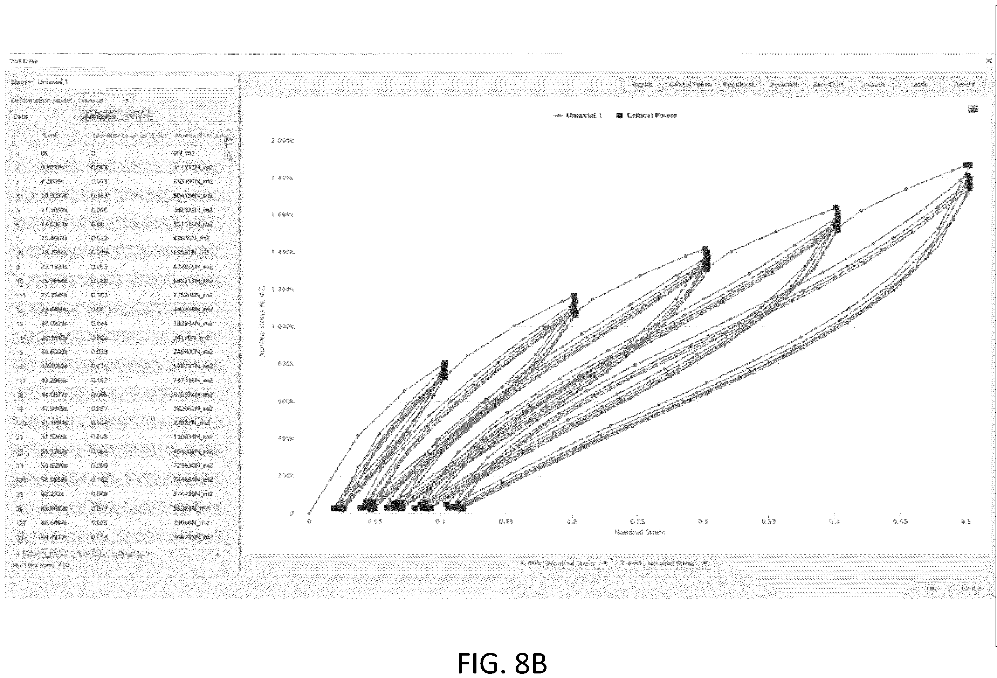

FIGS. 8A and 8B are screenshots that show a data set before decimation (FIG. 8A) and after decimation (FIG. 8B), where the critical points (e.g., identified by the critical points identifier 206) are preserved from FIG. 8A to FIG. 8B even though the data set is otherwise decimated. The decimation that occurs between the screenshots represented in FIG. 8A and FIG. 8B reduces the number of datapoints from over 4000 to about 400 points. Retaining the critical points, as shown, ensures that the reversals in the load history (e.g., stress and strain) are preserved. Thus, in a typical implementation, the computer 100, and design processes, enjoy the benefits of decimation (including processing efficiencies, for example) without sacrificing much, if anything, in terms of accuracy or usefulness of the data set in connection with material model calibration and/or subsequent design analysis utilizing the calibrated material model.

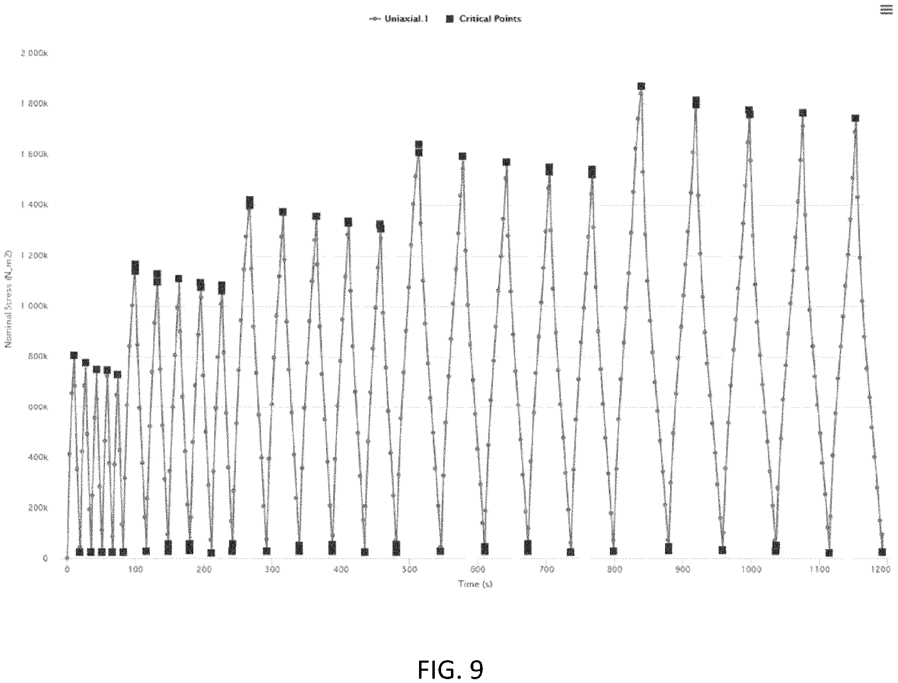

FIG. 9 shows a plot of nominal stress vs time in the data set that results from the decimator 208a performing decimation while preserving any critical points.

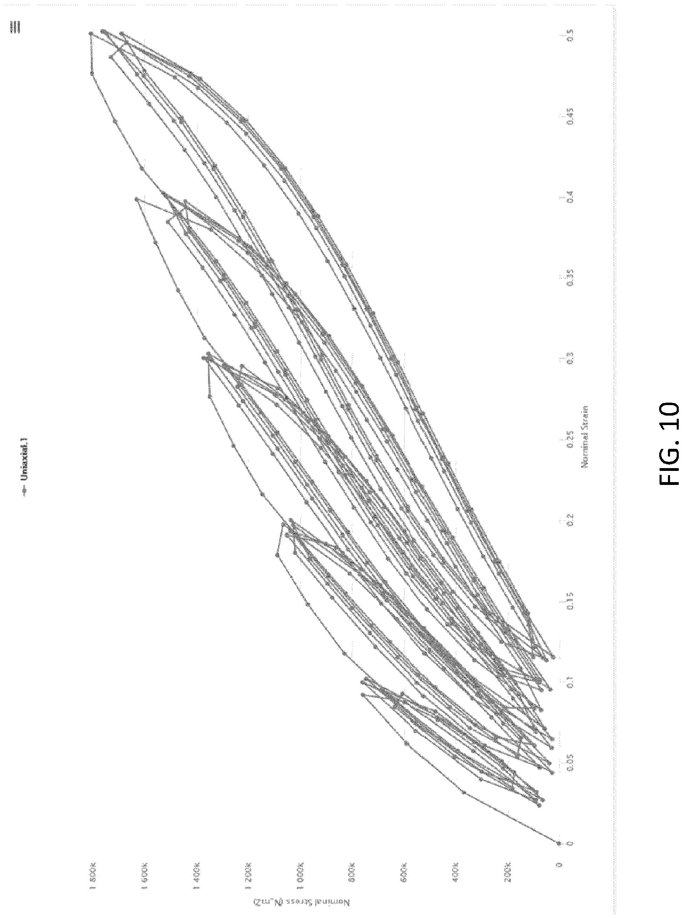

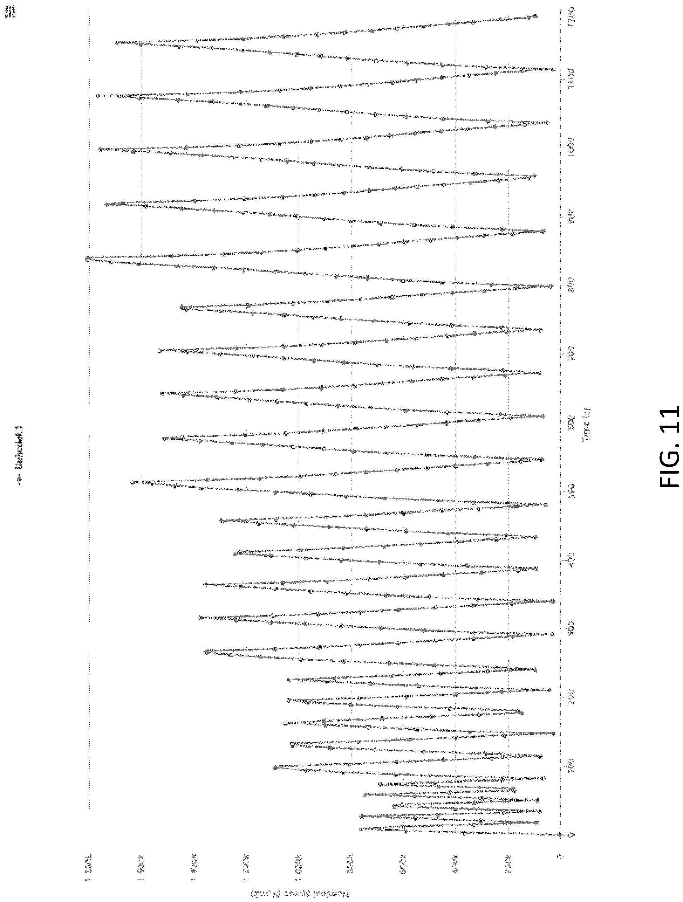

The results of preserving any critical points during decimation, which are shown in FIGS. 8A, 8B and 9, can be compared to results obtained from decimating without flagging or preserving critical points, in which case most of the datapoints representing local minima and maxima are deleted. An example of results obtained from decimating without flagging or preserving critical points is shown in FIGS. 10 and 11.

More specifically, FIG. 10 shows a plot of test data that has been decimated without flagging or preserving critical points. The plotted test data represents nominal strain (on the abscissa) and nominal stress (on the ordinate). As compared to the plot in FIG. 8B, for example, the plot in FIG. 10 makes clear that many, if not most, of the datapoints representing local minima and maxima are deleted when critical points are not flagged or preserved during decimating. FIG. 11, which shows a plot of nominal stress vs time test data that has been decimated without flagging or preserving critical points bolsters this point.

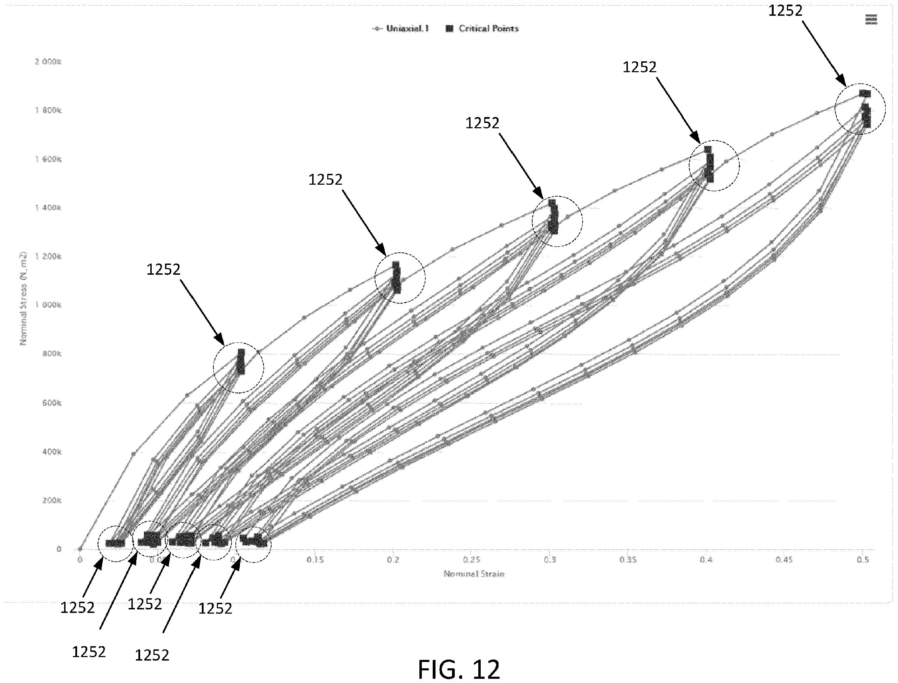

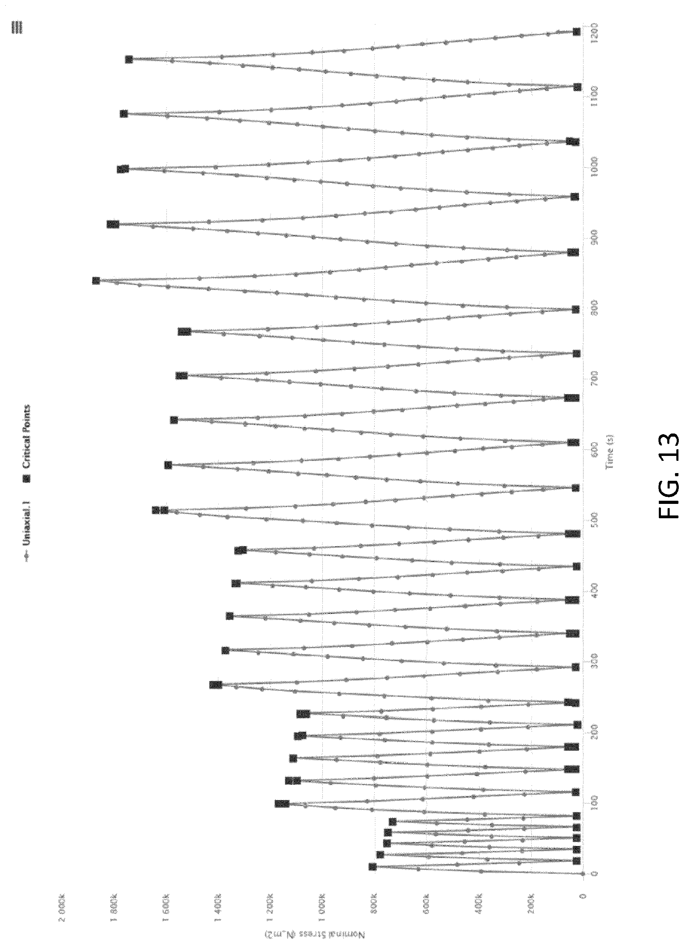

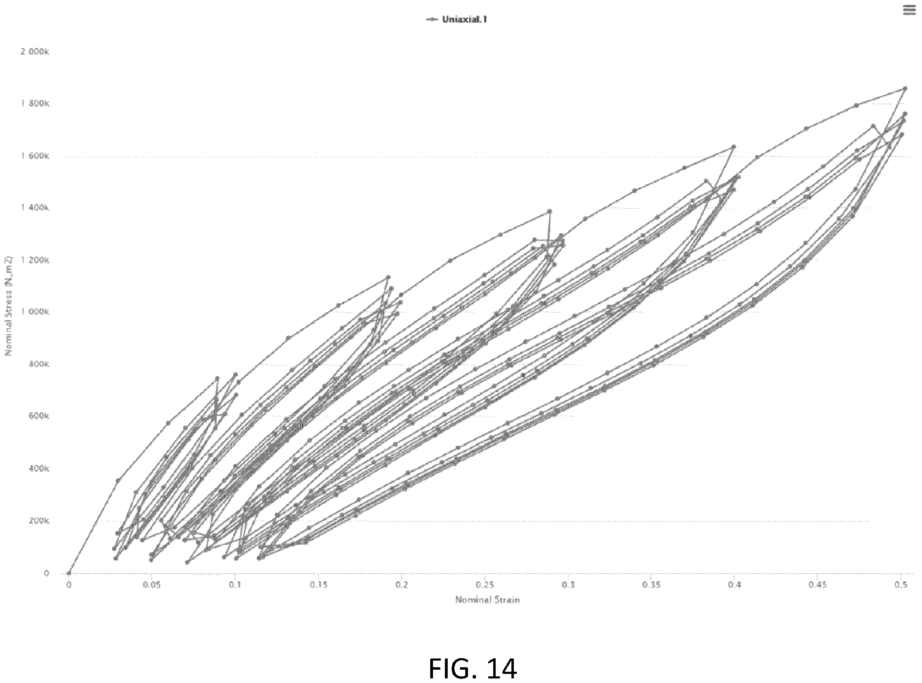

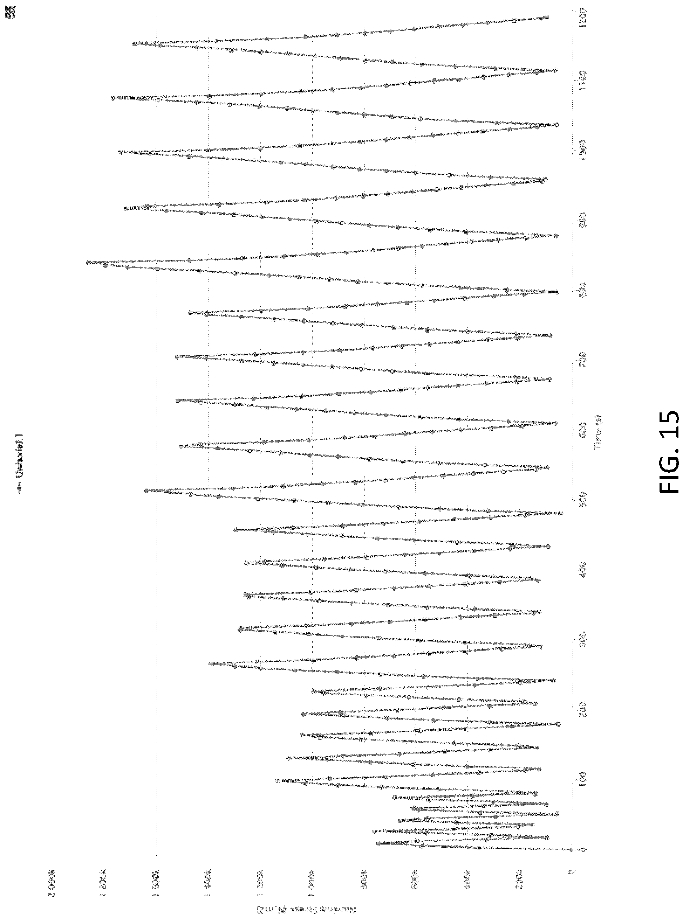

Similarly, when regularizing data with critical points, data is regularized over each interval running between flagged critical points. FIG. 12 and 13 are plots showing a data set that has been regularized, while preserving critical points (which are located within the circles 1252 in FIG. 12), to reduce the dataset from originally more than 4000 points to about 400 points. This result can be compared to regularizing without flagging critical points, which like decimation can, and generally does, result in the loss of local minima and maxima since these do not necessarily align with the times of the resampled datapoints in the regularization. (*See*, *e.g.,* FIG. 14 and 15).

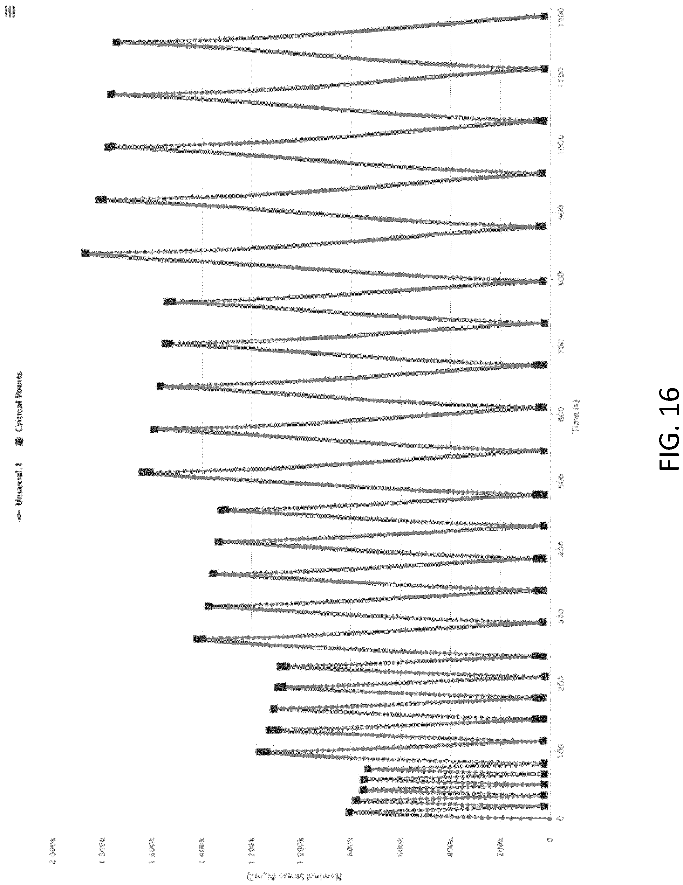

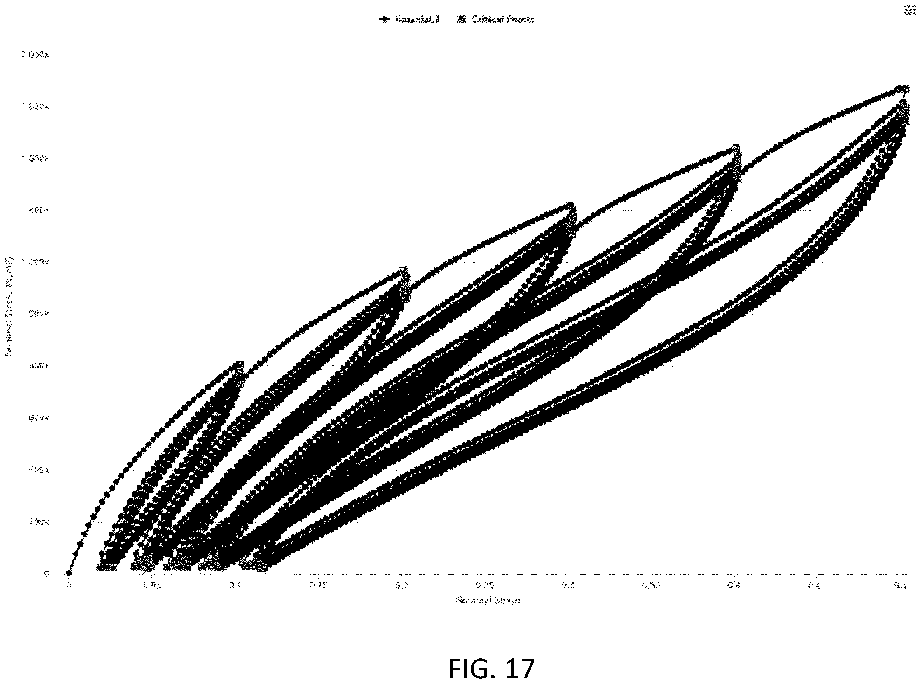

Similarly, smoothing the data with critical points results in the Savitsky-Golay algorithm utilized by the smoothing tool being applied over intervals running between critical points, while the critical points themselves are not modified. Additionally, the smoothing is performed in a manner to preserve C1 discontinuities at the critical points. In FIGS. 16 and 17, smoothing, with critical points preserved, has been applied to the stress and strain time histories, and, as can be seen, the local minima/maxima are retained.

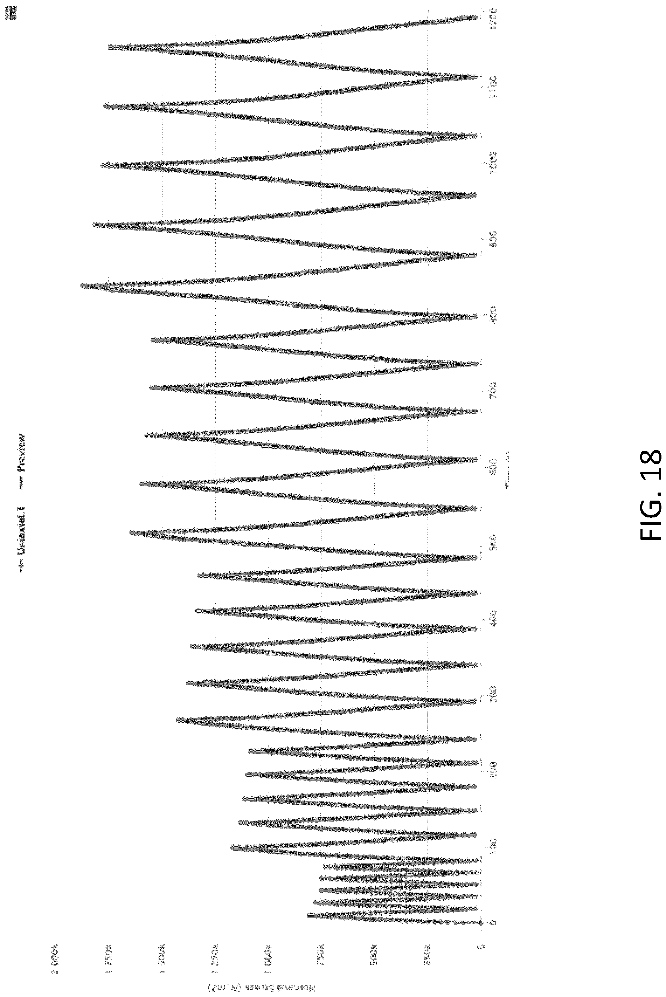

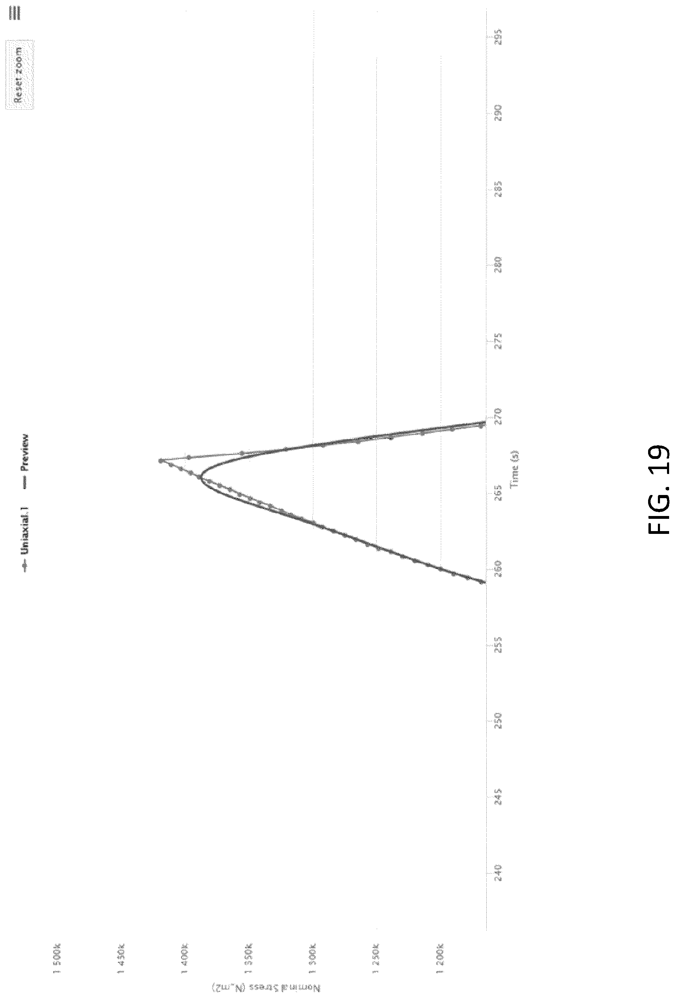

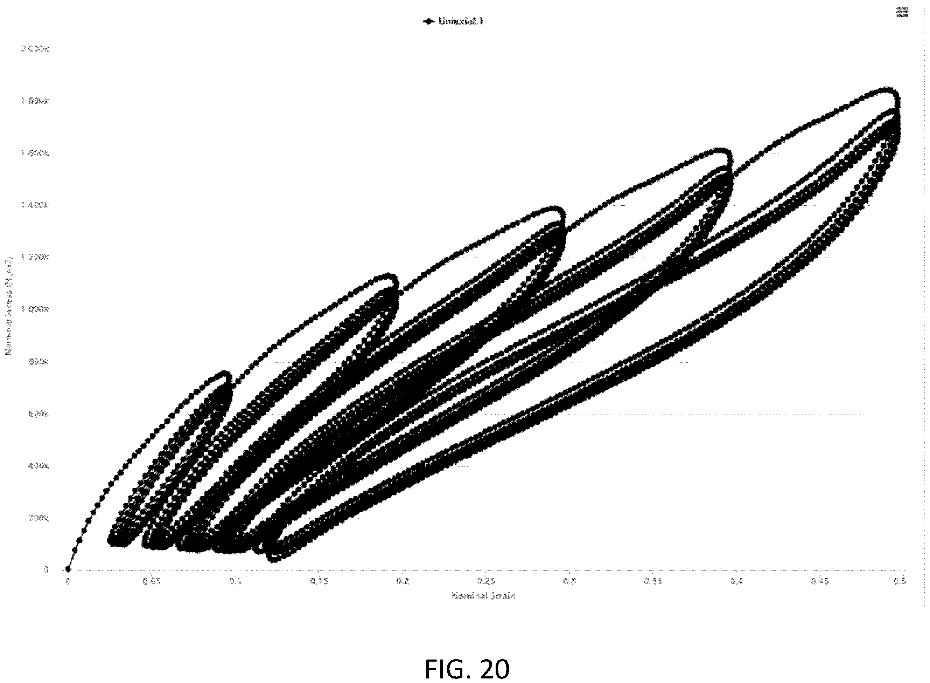

This is in contrast to smoothing the data without flagging and preserving critical points. Since these local minima/maxima represent sharp reversals (C1 discontinuities) that cannot be accurately approximated by the polynomial fitting inherent in the Savitsky-Golay smoothing algorithm, the result of smoothing is that these sharp sudden reversals are smoothed into gradual reversals and the values of the local minimal/maximal stresses and strains are changed. (See, e.g., FIGS. 18, 19, and 20).

A number of embodiments of the invention have been described. Nevertheless, it will be understood that various modifications may be made without departing from the scope of the invention.

The library of uncalibrated material models can include any number of a wide variety of material models, such as the Bergstrom-Boyce polymer hysteresis model, the Johnson-Holmquist & Johnson-Holmquist-Beissel ceramics and brittle materials material models, the Holmquist-Johnson-Cook concrete material model, a material model for tertiary creep analysis using the Omega method, a continuum damage material model for fabric reinforced composites, an isotropic porous viscoplasticity material model for metals, a material model for damage and failure in unidirectional fiber composite materials, a material model for multilinear kinematic hardening for cyclic plasticity, a material model for lead-free solder, an orthotropic honeycomb material model, a foam material model, a solder creep material model, an elastic-plastic behavior of shape memory alloy material model, and/or others.

This document refers throughout to a user taking various actions. Unless otherwise indicated, it should be understood that the user is a human taking action by interacting with a computer (e.g., 100) through one or more human machine interface (HMI) devices. Such devices can include, for example, one or more of a display screen (touch sensitive or otherwise), a keyboard, a computer mouse, a microphone, a speaker, and/or any one or more of a variety audible, visual, or tactile input-output devices. These HMI devices may be directly connected to the computer 100 via the I/O device interface 110 or across a network via the network interface 108. Other options for connectivity and human interaction with the computer are possible as well.

The test data can be stored in and handled by the computer in any number of ways. In an exemplary implementation, each test data set represents a single test data object. In some implementations, a "test data object" may contain multiple synchronized data series. Synchronized refers to the fact that, in a typical implementation, each data series in the test data object contains the same number of entries with each series representing a different quantity like time or frequency, stress, strain, force, displacement, temperature, etc. (there are many possible types of series that could exist). This can be thought of (and represented by) multiple columns in a table of data. The test data object, in some implementations, also includes metadata such as what sort of deformation state is being applied to a specimen, general text fields that could contain useful details about the test, such as lab temperature, date, test machine data, etc., and a "domain" tag to define what sort of data the test represents (e.g., whether the data represents various measurements over time or represents something about how the response changes with respect to a sweep over various excitation frequencies.

Critical points can be denoted in any number of a variety of ways. In an exemplary implementation, critical points are denoted to flag particular rows of data, for example, in a test data series. This flagging, once committed to the test data object typically persists with the test data object unless and until the user edits it. In some implementations, the tool for specifying critical points provides a function to automatically flag all reversals in a series as critical points. However, different, or more elaborate algorithms may be implemented instead. Additionally, in various implementations, the user has explicit manual control to specify individual data points as being critical or not. The identity of the critical points (however they are defined) generally becomes part of the test data object and modifies the way that various pre-processing algorithms operate to ensure these bits of the data series are preserved without modification.

A single test data object typically remains as a single test data object when critical points are defined - it is not split into multiple test data objects. Rather, in some implementations, the filters/preprocessing tools treat the data series contained in the test data object in a piecewise manner based on the defined critical points so as to preserve those points and any discontinuities they represent. Also, the filters/preprocessing algorithms generally do not split a test data object into multiple test data objects, although that is possible.

The test data preprocessors can be implemented in any number of a ways. In an exemplary implementation, one or more of the test data preprocessors is implemented as a kernel.

It should be understood that the example embodiments described herein may be implemented in many different ways. In some instances, the various methods and machines described herein may each be implemented by a physical, virtual, or hybrid general purpose computer, such as a computer system, or a computer network environment, such as those described herein. The computer / system may be transformed into the machines that execute the methods described herein, for example, by loading software instructions into either memory or non-volatile storage for execution by the CPU. One of ordinary skill in the art should understand that the computer / system and its various components may be configured to carry out any embodiments or combination of embodiments of the present invention described herein. Further, the system may implement the various embodiments described herein utilizing any combination of hardware, software, and firmware modules operatively coupled, internally, or externally, to or incorporated into the computer / system.

Various aspects of the subject matter disclosed herein can be implemented in digital electronic circuitry, or in computer-based software, firmware, or hardware, including the structures disclosed in this specification and/or their structural equivalents, and/or in combinations thereof. In some embodiments, the subject matter disclosed herein can be implemented in one or more computer programs, that is, one or more modules of computer program instructions, encoded on computer storage medium for execution by, or to control the operation of, one or more data processing apparatuses (e.g., processors). Alternatively, or additionally, the program instructions can be encoded on an artificially generated propagated signal, for example, a machine-generated electrical, optical, or electromagnetic signal that is generated to encode information for transmission to suitable receiver apparatus for execution by a data processing apparatus. A computer storage medium can be, or can be included within, a computer-readable storage device, a computer-readable storage substrate, a random or serial access memory array or device, or a combination thereof. While a computer storage medium should not be considered to be solely a propagated signal, a computer storage medium may be a source or destination of computer program instructions encoded in an artificially generated propagated signal. The computer storage medium can also be, or be included in, one or more separate physical components or media, for example, multiple CDs, computer disks, and/or other storage devices.

Certain operations described in this specification (e.g., aspects of those represented in FIGS. 3 and 6, and otherwise disclosed herein) can be implemented as operations performed by a data processing apparatus (e.g., a processor / specially programmed processor / computer) on data stored on one or more computer-readable storage devices or received from other sources, such as the computer system and/or network environment described herein. The term "processor" (or the like) encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application specific integrated circuit). The apparatus can also include, in addition to hardware, code that creates an execution environment for the computer program in question, for example, code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing, and grid computing infrastructures.

While this specification contains many specific implementation details, these should not be construed as limitations on the scope of any inventions or of what may be claimed, but rather as descriptions of features specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination, as long as they fall within the scope as defined by the appended claims.

Similarly, while operations may be described herein as occurring in a particular order or manner, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Other implementations are within the scope of the claims.

## Claims

1. A computer-implemented method comprising:
receiving (304), in computer memory, a first test data set that comprises results of a real-world test of a material, wherein the first test data set comprises a plurality of test data points;
identifying (308) one or more critical points among the test data points in the first test data set, wherein identifying one or more critical points among the test data points in the first test data set comprises:
identifying a data point in the first test data set for which an immediately preceding data point and an immediately following data point are both greater than or both less than the data point, or
identifying a data point in the first test data set for which one of an immediately preceding data point or an immediately following data point is equal and the other of the immediately preceding data point or the immediately following data point is not equal; and
processing the first test data set with a computer processor to produce a second test data set with differing test data points than the first test data set, wherein the second test data set includes all the test data points that were identified as critical points in the first test data set and at least some other data points.

2. The computer-implemented method of claim 1, further comprising:
calibrating a material model based on the second test data set to produce a calibrated material model,
wherein the material model is an equation with parameters, stored in a computer-readable medium, that describes a relationship between two or more characteristics of the tested material.

3. The computer-implemented method of claim 2, further comprising:
utilizing the calibrated material model to analyze a virtual design of a real-world product that includes the material.

4. The computer-implemented method of claim 1, wherein the processing of the first test data set comprises individually processing each respective one of a plurality of intervals in the first test data set, and
wherein each one of the intervals is defined by one or more of the critical points in the first test data set.

5. The computer-implemented method of claim 4, wherein:
a first one of the intervals is defined by an initial point in the first test data set and a first one of the critical points after the initial point,
a second one of the intervals is defined by a last one of the critical points before an end point in the first test data set and the end point in the first test data set, and/or
other ones of the intervals are defined by sequential critical points in the first data set.

6. The computer-implemented method of claim 5, wherein the processing comprises decimating each respective one of the intervals in the first test data set, regularizing each respective one of the intervals in the first test data set, and/or smoothing each respective one of the intervals in the first test data set.

7. The computer-implemented method of claim 4, further comprising:
mapping each of the critical points in the first data set to a corresponding one of the data points in the second data set.

8. The computer-implemented method of claim 7, further comprising flagging each respective one of the mapped data points in the second data set as critical.

9. The computer-implemented method of claim 1, further comprising:
after processing, returning the second test data set to a user interface for display,
wherein the second test data set is displayed on a computer screen in a manner that visually distinguishes the critical points from the other data points in the second test data set.

10. The computer-implemented method of claim 1, wherein the one or more critical points are identified automatically by a computer, the method further comprising:
enabling a user to add to or delete from the one or more critical points identified among the test data points in the first test data set prior to processing the first test data set.

11. A computer system comprising:
a computer processor; and
computer-based memory operatively coupled to the computer processor, wherein the computer-based memory stores computer-readable instructions that, when executed by the computer processor, cause the computer-based system to perform the method of any one of claims 1 to 10.

12. A computer program comprising computer-readable instructions that, when executed by a computer, cause the computer to carry out the method of any one of claims 1 to 10.

13. A non-transitory computer readable medium having stored thereon the computer program of claim 12.

## Patentansprüche

1. Computerimplementiertes Verfahren, umfassend:
Empfangen (304) eines ersten Testdatensatzes in einem Computerspeicher, der Resultate eines Realwelttests eines Materials umfasst, wobei der erste Testdatensatz eine Vielzahl von Testdatenpunkten umfasst;
Identifizieren (308) eines oder mehrerer kritischer Punkte unter den Testdatenpunkten in dem ersten Testdatensatz, wobei das Identifizieren eines oder mehrerer kritischer Punkte unter den Testdatenpunkten in dem ersten Testdatensatz Folgendes umfasst:
Identifizieren eines Datenpunkts in dem ersten Testdatensatz, für den ein unmittelbar vorangehender Datenpunkt und ein unmittelbar folgender Datenpunkt beide größer oder beide kleiner als der Datenpunkt sind, oder
Identifizieren eines Datenpunktes in dem ersten Testdatensatz, für den einer von einem unmittelbar vorangehenden Datenpunkt oder einem unmittelbar folgenden Datenpunkt gleich ist und der andere von dem unmittelbar vorangehenden Datenpunkt oder dem unmittelbar folgenden Datenpunkt nicht gleich ist; und
Verarbeiten des ersten Testdatensatzes mit einem Computerprozessor, um einen zweiten Testdatensatz mit anderen Testdatenpunkten als dem ersten Testdatensatz zu erzeugen, wobei der zweite Testdatensatz alle Testdatenpunkte, die in dem ersten Testdatensatz als kritische Punkte identifiziert wurden, und mindestens einige andere Datenpunkte enthält.

2. Computerimplementiertes Verfahren nach Anspruch 1, ferner umfassend:
Kalibrieren eines Materialmodells basierend auf dem zweiten Testdatensatz zum Erzeugen eines kalibrierten Materialmodells,
wobei das Materialmodell eine Gleichung mit Parametern ist, die in einem computerlesbaren Medium gespeichert ist und die ein Verhältnis zwischen zwei oder mehreren Eigenschaften des getesteten Materials beschreibt.

3. Computerimplementiertes Verfahren nach Anspruch 2, ferner umfassend:
Verwenden des kalibrierten Materialmodells zum Analysieren einer virtuellen Konstruktion eines Realweltprodukts, das das Material beinhaltet.

4. Computerimplementiertes Verfahren nach Anspruch 1, wobei das Verarbeiten des ersten Testdatensatzes ein individuelles Verarbeiten von jedem jeweiligen der Intervalle aus einer Vielzahl von Intervallen in dem ersten Testdatensatz umfasst, und
wobei jedes der Intervalle durch einen oder mehrere der kritischen Punkte in dem ersten Testdatensatz definiert ist.

5. Computerimplementiertes Verfahren nach Anspruch 4, wobei:
ein erstes der Intervalle durch einen Anfangspunkt in dem ersten Testdatensatz und einen ersten der kritischen Punkte nach dem Anfangspunkt definiert ist,
ein zweites der Intervalle durch einen letzten der kritischen Punkte vor einem Endpunkt in dem ersten Testdatensatz und den Endpunkt in dem ersten Testdatensatz definiert ist, und/oder
die anderen Intervalle durch aufeinanderfolgende kritische Punkte in dem ersten Datensatz definiert sind.

6. Computerimplementiertes Verfahren nach Anspruch 5, wobei das Verarbeiten ein Dezimieren von jedem jeweiligen der Intervalle in dem ersten Testdatensatz, ein Regularisieren von jedem jeweiligen der Intervalle in dem ersten Testdatensatz und/oder ein Glätten von jedem jeweiligen der Intervalle in dem ersten Testdatensatz umfasst.

7. Computerimplementiertes Verfahren nach Anspruch 4, ferner umfassend:
Zuordnen von jedem der kritischen Punkte in dem ersten Datensatz zu einem entsprechenden Datenpunkt in dem zweiten Datensatz.

8. Computerimplementiertes Verfahren nach Anspruch 7, ferner umfassend ein Markieren als kritisch von jedem einzelnen der abgebildeten Datenpunkte in dem zweiten Datensatz.

9. Computerimplementiertes Verfahren nach Anspruch 1, ferner umfassend:
nach Verarbeiten, Zurückgeben des zweiten Testdatensatzes an eine Benutzerschnittstelle zur Anzeige,
wobei der zweite Testdatensatz auf eine Weise auf einem Computerbildschirm angezeigt wird, die die kritischen Punkte visuell von den anderen Datenpunkten in dem zweiten Testdatensatz unterscheidet.

10. Computerimplementiertes Verfahren nach Anspruch 1, wobei der eine oder die mehreren kritischen Punkte automatisch von einem Computer identifiziert werden, das Verfahren ferner umfassend:
Ermöglichen eines Hinzufügens oder Löschens von einem oder mehreren kritischen Punkten, die unter den Testdatenpunkten in dem ersten Testdatensatz identifiziert wurden, durch einen Benutzer vor Verarbeiten des ersten Testdatensatzes.

11. Computersystem, umfassend:
einen Computerprozessor, und
computergestützter Speicher, der funktionsfähig mit dem Computerprozessor gekoppelt ist, wobei der computergestützte Speicher computerlesbare Anweisungen speichert, die, wenn sie von dem Computerprozessor ausgeführt werden, das computergestützte System veranlassen, das Verfahren nach einem der Ansprüche 1 bis 10 durchzuführen.

12. Computerprogramm, umfassend computerlesbare Anweisungen, die, wenn sie von einem Computer ausgeführt werden, den Computer veranlassen, das Verfahren nach einem der Ansprüche 1 bis 10 durchzuführen.

13. Nicht-übertragbares computerlesbares Medium, auf dem das Computerprogramm nach Anspruch 12 gespeichert ist.

## Revendications

1. Procédé mis en œuvre par ordinateur, comprenant :
la réception (304), dans une mémoire informatique, d'un premier ensemble de données d'essai qui comprend les résultats d'un essai en conditions réelles d'un matériau, le premier ensemble de données d'essai comprenant une pluralité de points de données d'essai ;
l'identification (308) d'un ou plusieurs points critiques parmi les points de données d'essai dans le premier ensemble de données d'essai, l'identification d'un ou plusieurs points critiques parmi les points de données d'essai dans le premier ensemble de données d'essai comprenant :
l'identification d'un point de données dans le premier ensemble de données d'essai pour lequel un point de données immédiatement précédent et un point de données immédiatement suivant sont tous deux supérieurs ou tous deux inférieurs au point de données, ou
l'identification d'un point de données dans le premier ensemble de données d'essai pour lequel l'un parmi un point de données immédiatement précédent ou un point de données immédiatement suivant est égal et l'autre parmi le point de données immédiatement précédent ou le point de données immédiatement suivant n'est pas égal ; et
le traitement du premier ensemble de données d'essai avec un processeur informatique pour produire un deuxième ensemble de données d'essai avec des points de données d'essai différents du premier ensemble de données d'essai, le deuxième ensemble de données d'essai comprenant tous les points de données d'essai qui ont été identifiés comme des points critiques dans le premier ensemble de données d'essai et au moins certains autres points de données.

2. Procédé mis en œuvre par processeur selon la revendication 1, comprenant en outre :
l'étalonnage d'un modèle de matériau sur la base du deuxième ensemble de données d'essai pour produire un modèle de matériau étalonné,
dans lequel le modèle de matériau est une équation avec des paramètres, stockée dans un support lisible par ordinateur, qui décrit une relation entre au moins deux caractéristiques du matériau soumis à essai.

3. Procédé mis en œuvre par processeur selon la revendication 2, comprenant en outre :
l'utilisation du modèle de matériau étalonné pour analyser une conception virtuelle d'un produit du monde réel qui comprend le matériau.

4. Procédé mis en œuvre par ordinateur selon la revendication 1, dans lequel le traitement du premier ensemble de données d'essai comprend le traitement individuel de chaque intervalle respectif d'une pluralité d'intervalles dans le premier ensemble de données d'essai, et
dans lequel chacun des intervalles est défini par un ou plusieurs des points critiques dans le premier ensemble de données d'essai.

5. Procédé mis en œuvre par ordinateur selon la revendication 4, dans lequel :
un premier intervalle parmi les intervalles est défini par un point initial dans le premier ensemble de données d'essai et un premier point critique parmi les points critiques après le point initial,
un deuxième intervalle parmi les intervalles est défini par un dernier point critique parmi les points critiques avant un point final dans le premier ensemble de données d'essai et le point final dans le premier ensemble de données d'essai, et/ou
les autres intervalles parmi les intervalles sont définis par des points critiques séquentiels dans le premier ensemble de données.

6. Procédé mis en œuvre par ordinateur selon la revendication 5, dans lequel le traitement comprend le sous-échantillonnage de chacun des intervalles respectifs dans le premier ensemble de données d'essai, la régularisation de chacun des intervalles respectifs dans le premier ensemble de données d'essai, et/ou le lissage de chacun des intervalles respectifs dans le premier ensemble de données d'essai.

7. Procédé mis en œuvre par processeur selon la revendication 4, comprenant en outre :
la mise en correspondance de chacun des points critiques dans le premier ensemble de données avec un point de données correspondant du deuxième ensemble de données.

8. Procédé mis en œuvre par ordinateur selon la revendication 7, comprenant en outre le marquage de chacun des points de données mis en correspondance respectifs dans le deuxième ensemble de données comme étant critiques.

9. Procédé mis en œuvre par processeur selon la revendication 1, comprenant en outre :
après traitement, le renvoi du deuxième ensemble de données d'essai à une interface utilisateur pour affichage,
le deuxième ensemble de données d'essai étant affiché sur un écran d'ordinateur d'une manière qui distingue visuellement les points critiques des autres points de données dans le deuxième ensemble de données d'essai.

10. Procédé mis en œuvre par ordinateur selon la revendication 1, dans lequel les un ou plusieurs points critiques sont identifiés automatiquement par un ordinateur, le procédé comprenant en outre :
l'autorisation à un utilisateur d'ajouter ou de supprimer des points critiques dans les un ou plusieurs points critiques identifiés parmi les points de données d'essai dans le premier ensemble de données d'essai avant de traiter le premier ensemble de données d'essai.

11. Système informatique comprenant :
un processeur informatique ; et
une mémoire informatique couplée fonctionnellement au processeur informatique, la mémoire informatique stockant des instructions lisibles par ordinateur qui, lorsqu'elles sont exécutées par le processeur informatique, amènent le système informatique à réaliser le procédé selon l'une quelconque des revendications 1 à 10.

12. Programme informatique comprenant des instructions lisibles par ordinateur qui, lorsqu'elles sont exécutées par un ordinateur, amènent l'ordinateur à réaliser le procédé selon l'une quelconque des revendications 1 à 10.

13. Support lisible par ordinateur non transitoire sur lequel est stocké le programme informatique selon la revendication 12.
